# EUROPEAN PATENT SPECIFICATION

(11) **EP 2 561 066 B1**
(45) Date of publication and mention of the grant of the patent: **30.03.2016**
(21) Application number: 11730426.1
(22) Date of filing: 21.04.2011
(51) Int. Cl.: C12N 5/073

(54) **ADHERENT STROMAL CELLS DERIVED FROM PLANCENTAS OF MULTIPLE DONORS AND USES THEREOF**
ADHERENTE STROMAZELLEN AUS PLAZENTEN MULTIPLER SPENDER UND DEREN VERWENDUNG
CELLULES STROMALES ADHÉRENTES ISSUES DE PLACENTAS DE DONNEURS MULTIPLES ET LEURS UTILISATIONS

(30) Priority: 23.04.2010 US 327330 P
(43) Date of publication of application: 27.02.2013
(73) Proprietor: Pluristem Ltd., 31905 Haifa (IL)
(72) Inventor: ABERMAN, Zami, 40600 Tel-Mond (IL)
(74) Representative: Dörries, Hans Ulrich
(86) International application number: PCT/IB2011/001413
(87) International publication number: WO 2011/132087

(56) References cited:
- WO-A1-2009/037690
- WO-A1-2009/144720
- MAGATTI MARTA ET AL: "Human amnion mesenchyme harbors cells with allogeneic T-cell suppression and stimulation capabilities.", STEM CELLS (DAYTON, OHIO) JAN 2008 LNKD- PUBMED:17901399, vol. 26, no. 1, January 2008 (2008-01), pages 182-192, XP9152190, ISSN: 1549-4918
- PRATHER WILLIAM R ET AL: "The role of placental-derived adherent stromal cell (PLX-PAD) in the treatment of critical limb ischemia.", CYTOTHERAPY 2009 LNKD- PUBMED:19526389, vol. 11, no. 4, 2009, pages 427-434, XP9127935, ISSN: 1477-2566
- PRATHER WILLIAM R ET AL: "Placental-derived and expanded mesenchymal stromal cells (PLX-I) to enhance the engraftment of hematopoietic stem cells derived from umbilical cord blood.", EXPERT OPINION ON BIOLOGICAL THERAPY AUG 2008 LNKD- PUBMED:18613774, vol. 8, no. 8, August 2008 (2008-08), pages 1241-1250, XP9128193, ISSN: 1744-7682
- LEE PO-CHANG ET AL: "Effectiveness of an organ-sharing program in providing zero HLA-A,B,DR mismatched kidneys for transplantation in Taiwan", JOURNAL OF THE FORMOSAN MEDICAL ASSOCIATION, vol. 99, no. 6, June 2000 (2000-06), pages 447-452, XP9152194, ISSN: 0929-6646
- TIERCY J M: "Molecular basis of HLA polymorphism: implications in clinical transplantation.", TRANSPLANT IMMUNOLOGY MAY 2002 LNKD- PUBMED:12180827, vol. 9, no. 2-4, May 2002 (2002-05), pages 173-180, XP27388192, ISSN: 0966-3274
- PAROLINI ORNELLA ET AL: "Concise review: isolation and characterization of cells from human term placenta: outcome of the first international Workshop on Placenta Derived Stem Cells.", STEM CELLS (DAYTON, OHIO) FEB 2008 LNKD- PUBMED:17975221, vol. 26, no. 2, February 2008 (2008-02), pages 300-311, XP2567088, ISSN: 1549-4918

## Description

### INTRODUCTION

Pharmaceutical compositions comprising adherent stromal cells (ASCs) are provided. The ASCs are obtained from at least two placentas. Articles of manufacture comprising the pharmaceutical compositions together with a delivery device for administering the ASCs to a subject are also provided. Also provided are methods of treating various diseases and conditions that are treatable by administering ASCs to a subject in need of treatment.

In recent years, considerable activity has focused on the therapeutic potential of mesenchymal stromal cells (MSCs) for various medical applications including tissue repair of damaged organs such as the brain, heart, bone and liver and in support of bone marrow transplantations (BMT). MSCs, a heterogeneous population of cells obtained from, for example, bone marrow, adipose tissue, placenta, or blood, are capable of differentiating into different types of mesenchymal mature cells (e.g. reticular endothelial cells, fibroblasts, adipocytes, osteogenic precursor cells) depending upon influences from various bioactive factors. Accordingly, MSCs have been widely studied in regenerative medicine as the foundation to build new tissues such as bone, cartilage and fat for the repair of injury or replacement of pathologic tissues and as treatment for genetic and acquired diseases [Fibbe and Noort, Ann N Y Acad Sci (2003) 996: 235-44; Horwitz et al., Cytotherapy (2005) 7(5): 393-5; Zimmet and Hare, Basic Res Cardiol (2005) 100(6): 471-81]. Furthermore, the multi potent ability of MSCs, their easy isolation and culture, as well as their high *ex vivo* expansion potential make them an attractive therapeutic tool [Fibbe and Noort, supra; Minguell et al. Exp Biol Med (Maywood) (2001) 226(6): 507-20].

Placental derived MSCs exhibit many markers common to MSCs isolated from other tissues, for example, CD105, CD73, CD90 and CD29, and the lack of expression of hematopoietic, endothelial and trophoblastic-specific cell markers. Adipogenic, osteogenic, and neurogenic differentiation have been achieved after culturing placental derived MSCs under appropriate conditions [Yen et al., Stem Cells (2005) 23(1): 3-9]. Furthermore, MSCs isolated from placenta and cultured *in vitro* have been demonstrated to be immune privileged in a similar fashion as bone marrow derived MSCs. Thus, the placenta provides an ethically non-controversial and easily accessible source of MSCs for experimental and clinical applications [Zhang et al., Exp Hematol (2004) 32(7): 657-64].

Methods of making ASCs and using them to treat various conditions are described in International Application No. PCT/IL2008/001185 (published as WO 2009/037690 A1) and International Application No. PCT/IL2009/000527 (published as WO/2009/144720). Those applications describe derivation of ASCs from a single placenta obtained from a single allogeneic donor for transplantation into a subject. As described herein, the inventors have now determined that it is possible to obtain ASCs from multiple placentas, create a mixed ASC population containing ASCs having different HLA types derived from at least two placentas, and then transplant that mixed population into a recipient. This finding, among others, makes it possible to manufacture ASCs by pooling placentas or cells derived from placentas and in that way provides new and useful manufacturing processes and new and useful cell compositions for therapeutic applications, among other things.

### SUMMARY

Provided are methods of treating at least one condition that can be treated by administration of placental-derived adherent stromal cells (ASCs) to a subject in need thereof. In some embodiments the methods include administering to the subject an effective amount of adherent stromal cells (ASCs), wherein the administered ASCs comprise ASCs from at least two donor placentas. In some embodiments, the method comprises administering to a subject an effective amount of ASCs, wherein the ASCs are prepared from at least two donor placentas. In some embodiments the ASCs are obtained by a method comprising culturing placental-derived cells in a three-dimensional (3D) culture. In some embodiments the 3D culturing comprises culturing in a 3D bioreactor. In some embodiments cells in the 3D bioreactor are cultured under perfusion. In some embodiments the 3D bioreactor comprises at least one adherent material selected from a polyester and a polypropylene. In some embodiments the 3D culturing occurs for at least three days. In some embodiments the 3D culture step occurs until at least 10 % of the cells are proliferating. In some embodiments the ASCs are positive for at least one marker selected from CD73, CD90, CD29, D7-FIB and CD105. In some embodiments the ASCs from each of the at least two donors are positive for the at least one marker. In some embodiments the ASCs are negative for at least one marker selected from CD3, CD4, CD45, CD80, HLA-DR, CD11b, CD14, CD19, CD34, CD200, KDR, CD31 and CD79. In some embodiments the ASCs from each of the at least two donors are negative for the at least one marker. In some embodiments the ASCs are PLX or PLX-C cells. In some embodiments the ASCs are obtained by a method comprising culturing placental-derived cells in a two-dimensional (2D) culture.

In some embodiments of the methods the at least one condition is selected from stem cell deficiency, heart disease, a neurodegenerative disorder, cancer, stroke, burns, loss of tissue, loss of blood, anemia, an autoimmune disease, ischemia, skeletal muscle regeneration, neuropathic pain, a compromised hematopoietic system, geriatric diseases, and a medical condition requiring connective tissue regeneration and/or repair. In some embodiments the neurodegenerative disorder is selected from multiple sclerosis (MS), Alzheimer's disease, and Parkinson's disease. In some embodiments the ischemia is peripheral arterial disease (PAD). In some embodiments the PAD is critical limb ischemia (CLI). In some embodiments the ischemia comprises ischemia of the central nervous system (CNS). In some embodiments the ischemia is selected from peripheral arterial disease, ischemic vascular disease, ischemic heart disease, ischemic brain disease, ischemic renal disease and ischemic placenta. In some embodiments, the compromised hematopoietic system is caused by radiation or by chemotherapy. In some embodiments the connective tissue comprises at least one of tendon, bone and ligament. In some embodiments the medical condition requiring connective tissue regeneration and repair is selected from bone fracture, bone cancer, burn wound, articular cartilage defect and deep wound. In some embodiments the medical condition requiring connective tissue regeneration and repair is selected from a subchondral-bone cyst, a bone fracture, an osteoporosis, an osteoarthritis, a degenerated bone, a cancer, a cartilage damage, an articular cartilage defect, a degenerative disc disease, an osteogenesis imperfecta (OI), a burn, a burn wound, a deep wound, a delayed wound-healing, an injured tendon and an injured ligament. In some embodiments the autoimmune disease is selected from rhumatoid arthritis, ankylosing spondylitis, inflammatory bowel disease (IBD), MS, diabetes type I, Goodpasture's syndrome, Graves' disease, Hashimoto's disease, Lupus, Myasthenia Gravis, Psoriasis, and Sjorgen's syndrome. In some embodiments the IBD is selected from Crohn's disease and ulcerative colitis.

In some embodiments of the methods the administered ASCs from at least two donors comprise ASCs from at least three, at least four, at least five, at least ten, at least twenty-five, or at least 100 donor placentas. In some embodiments the at least two donors have at least two different HLA genotypes. In some embodiments the at least two different HLA genotypes are genotypes of at least one of the HLA-A, HLA-B, HLA-DR, and HLA-DQ loci. In some embodiments the ASCs from at least two donors are administered to the subject from at least one aliquot. In some embodiments, all of the at least one aliquots comprise ASCs prepared from each of the at least two donors. In some embodiments, one or more of the at least one aliquots comprise ASCs prepared from each of the at least two donors. In some embodiments, one or more of the at least one aliquots comprise ASCs prepared from more than one donor. In some embodiments the aliquot comprising ASCs from each of the at least two donors or the aliquot comprising ASCs from more than one donor is made by a method comprising at least one step selected from mixing placental-derived cells prior to culturing *in vitro,* mixing placental-derived cells during 2D culturing, mixing placental-derived cells after 2D culturing, mixing placental-derived cells during 3D culturing, and mixing placental-derived cells after 3D culturing. In some embodiments the ASCs from at least two donors are administered to the subject from aliquots each comprising ASCs from only a single donor. In those embodiments in which aliquots comprising ASCs from only a single donor are administered to the patient, ASCs from the aliquot comprising ASCs from one donor may be administered concurrently with, within less than one hour after, within less than 6 hours after, within less than 12 hours after, or within less than 24 hours after ASCs from an aliquot comprising ASCs from any other donor are administered to that patient. In some embodiments the ASCs are administered in one or more treatment courses. In some embodiments, the ASC are administered as one treatment course, two treatment courses, not more than ten treatment courses, ten or more treatment courses, or treatment courses that continue throughout the life of the subject. One treatment course may be separated from another treatment course by 1 day, 2 days, 3 days, 4 days, 5 days, 1 week, 2 weeks, 3 weeks, 1 month, 2 month, 3 months, 4 months, 5 months, 6 months, 1 year, or by 2 or more years. In some embodiments, one treatment course comprises delivering from 1 to 40, from 5 to 40, from 10 to 30, about 5, about 10, about 15, about 20, about 25, about 30, about 35, about 40, or about 45, or about 50 separate injections to the subject. In some embodiments about 30 to about 50 injections of ASCs are administered to the subject for one or two treatment courses. In some embodiments the subject is in need of treatment for critical limb ischemia and the ASCs are administered to the subject in about 30 to about 50 intramuscular injections for one or two treatment courses.

Also provided are pharmaceutical compositions comprising ASCs. In some embodiments the pharmaceutical composition comprises ASCs from at least two donor placentas and a pharmaceutically acceptable carrier. In some embodiments the ASCs are obtained by a method comprising culturing placental-derived cells in a three-dimensional (3D) culture. In some embodiments the 3D culturing comprises culturing in a 3D bioreactor. In some embodiments cells in the 3D bioreactor are cultured under perfusion. In some embodiments the 3D bioreactor comprises at least one adherent material selected from a polyester and a polypropylene. In some embodiments the 3D culturing occurs for at least three days. In some embodiments the 3D culture step occurs until at least 10 % of the cells are proliferating. In some embodiments the ASCs are positive for at least one marker selected from CD73, CD90, CD29, D7-FIB and CD105. In some embodiments the ASCs from each of the at least two donors are positive for the at least one marker. In some embodiments the ASCs are negative for at least one marker selected from CD3, CD4, CD45, CD80, HLA-DR, CD11b, CD14, CD19, CD34, CD200, KDR, CD31 and CD79. In some embodiments the ASCs from each of the at least two donors are negative for the at least one marker. In some embodiments the ASCs are PLX or PLX-C cells. In some embodiments the ASCs comprise ASCs from at least three, at least four, at least five, at least ten, at least twenty-five, or at least 100 donors. In some embodiments the at least two donors have at least two different HLA genotypes. In some embodiments the at least two different HLA genotypes are genotypes of at least one of the HLA-A, HLA-B, HLA-DR, and HLA-DQ loci. In some embodiments the ASCs are obtained by a method comprising culturing placental-derived cells in a two-dimensional (2D) culture. In some embodiments the pharmaceutically acceptable carrier is an isotonic solution. In some embodiments the isotonic solution further comprises about 5% human serum albumin. In some embodiments the isotonic solution further comprises about 5% to about 10% dimethyl sulphoxide.

Also provided are articles of manufacture comprising one of the pharmaceutical compositions comprising ASCs and a delivery device for administering the ASCs to a subject. Sometimes the pharmaceutical composition is packaged within the delivery device. Sometimes the delivery device is suitable for administering the pharmaceutical composition by intravenous, intramuscular or subcutaneous injection.

It is to be understood that both the foregoing general description and the following detailed description are exemplary and explanatory only and are not restrictive of the invention, as claimed.

### BRIEF DESCRIPTION OF THE DRAWINGS

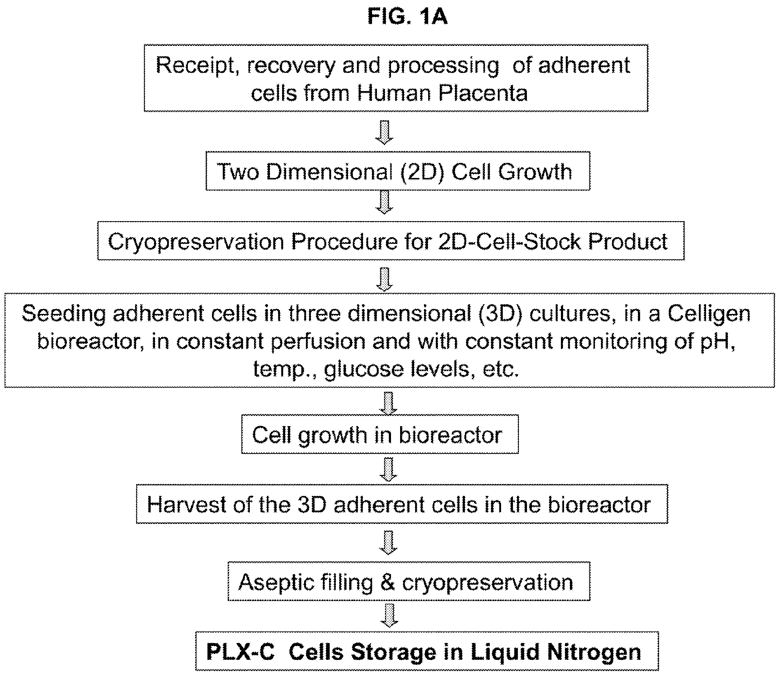

FIG. 1A is a flow chart depicting production of 3D adherent cell from placentas by Celligen^{™} (designated PLX-C cells).

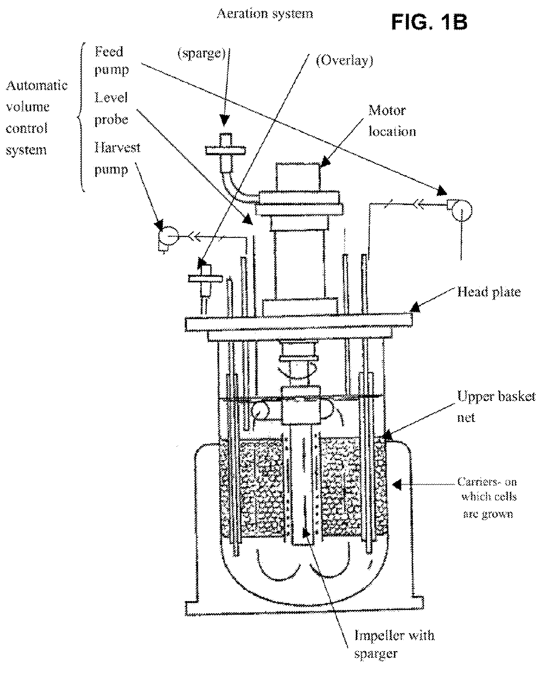

FIG. 1B is a diagram of a Celligen^{™} bioreactor vessel and ports adapted from The New Brunswick Scientific web site.

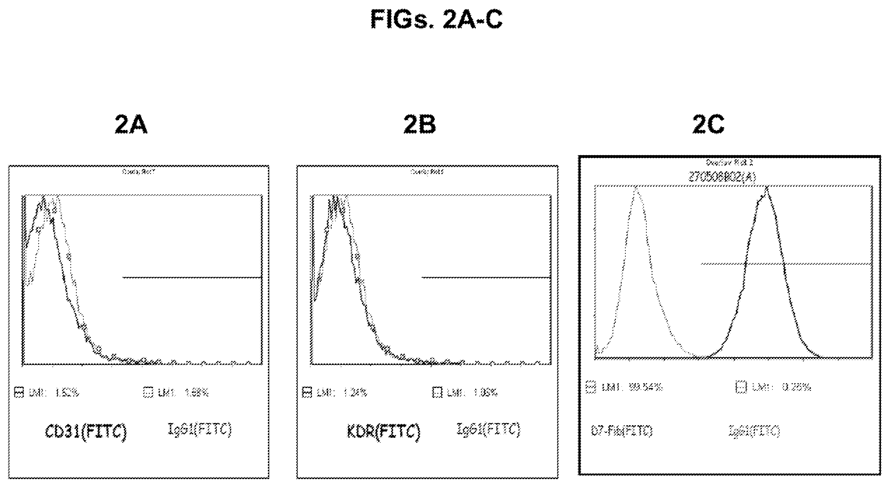

FIGs. 2A-C depict expression of fibroblast-typical markers but not expression of endothelial typical markers on PLX-C. Figure 2A depicts negative expression of the endothelial marker CD31; Figure 2B depicts negative expression of the endothelial marker KDR; and Figure 2C depicts positive expression of the human fibroblast marker (D7-FIB). Of note, the histograms shown in the grey/non-bold lines for Isotype IgG1 (FITC) represent the negative control while the histograms shown in the bold lines represent the positively stained cells.

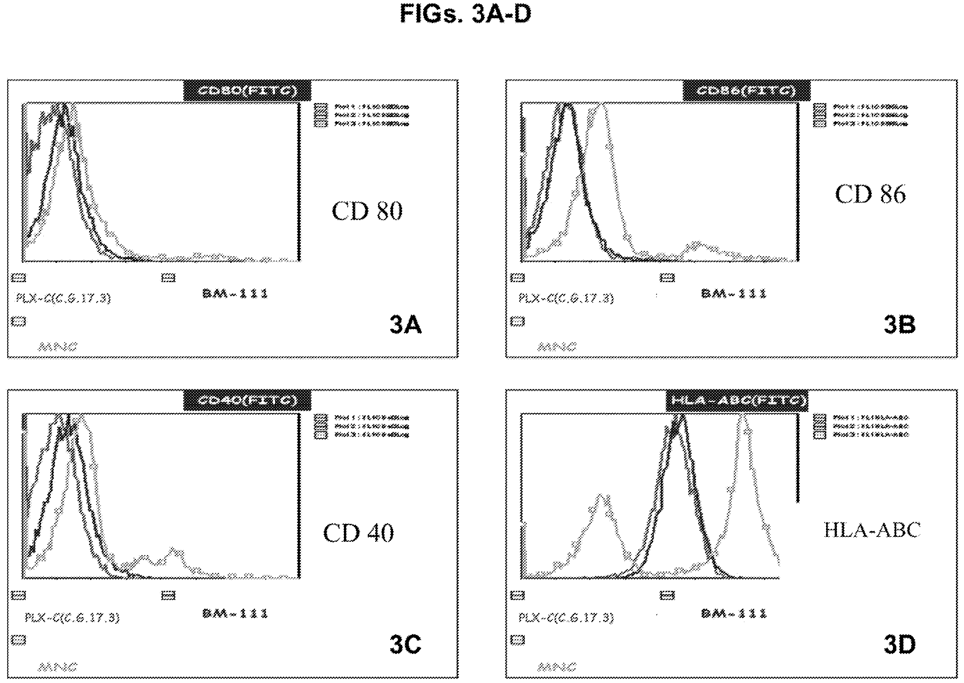

FIGs. 3A-D depict expression of stimulatory and co-stimulatory molecules on PLX-C cells. Figure 3A depicts PLX-C expression of CD80; Figure 3B depicts PLX-C expression of CD86; Figure 3C depicts PLX-C expression of CD40; and Figure 3D depicts PLX-C expression of HLA-A/B/C. Negative controls were prepared with relevant isotype fluorescence molecules. Of note, histograms shown in the dark grey lines indicate PLX-C marker-expressing population of cells, histograms shown in the bold/black lines indicate bone marrow (BM) marker-expressing population of cells, and histograms shown in the light grey lines indicate mononuclear cell (MNC) marker expressing population of cells.

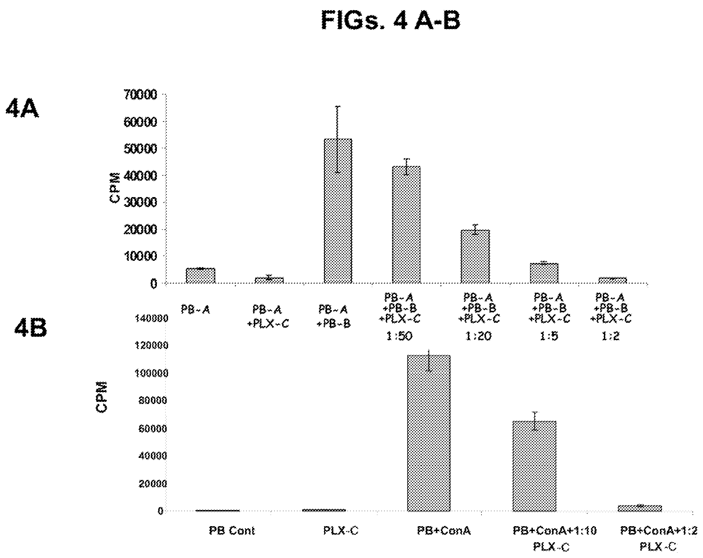

FIGs. 4A-B depict inhibition of lymphocyte proliferation by PLX-C. Figure 4A depicts MLR tests performed with 2 x 10⁵ peripheral blood (PB) derived MNC (donor A) stimulated with equal amount of irradiated (3000 Rad) PB derived MNCs (donor B) followed by addition of increasing amounts of PLX-C cells to the cultures. Three replicates of each group were seeded in 96-well plates. Proliferation rate was measured by [3H]thymidine incorporation; Figure 4B depict peripheral blood (PB) derived MNCs stimulated with ConA (1.5 mg /ml). Increasing amounts of PLX-C cells were added to the cultures. Three replicates of each group were seeded in 96-well plates. Proliferation rate was mesured by [3H]thymidine incorporation.

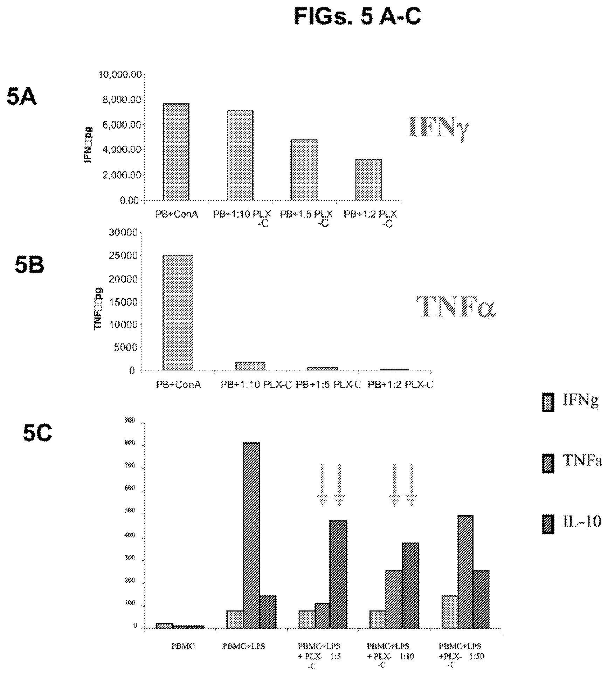

FIGs. 5A-C depict PLX-C regulation of pro-inflammatory and anti-inflammatory cytokine secretion following co-culture with peripheral blood cells. Figures 5A-B depict secretion of IFNγ (Figure 5A) and TNFα (Figure 5B) following co-culture of human derived MNCs (isolated from peripheral blood) stimulated with ConA with PLX-C; Figure 5C depicts secretion of IFNγ (left bar), TNFα (middle bar) and IL-10 (right bar) following co-culture of human derived MNCs (isolated from peripheral blood) stimulated with LPS with PLX-C. Supernatants were collected and subjected to cytokines analysis using ELISA.

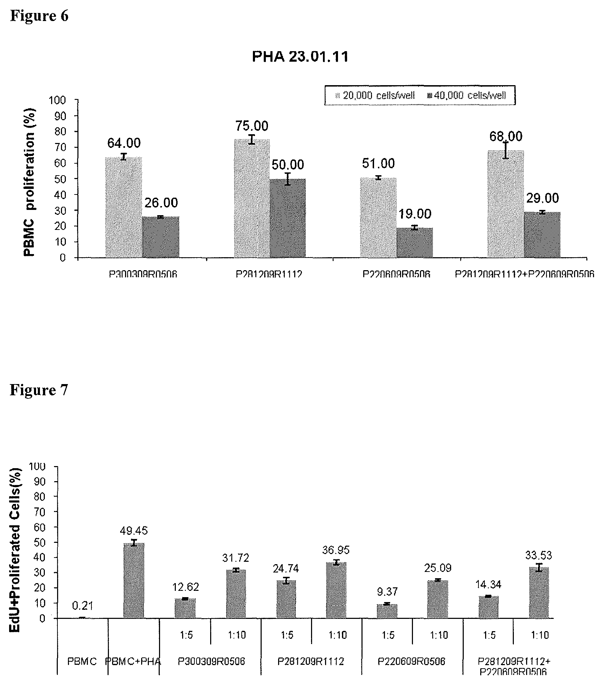

FIG. 6. depicts the average PBMC proliferation rate per bioreactors ± SD.
FIG. 7 depicts the average PBMC proliferation per bioreactors ± SD.

### DETAILED DESCRIPTION

Example 1 describes methods used to make placenta-derived adherent stromal cells (ASCs). As shown in Example 2, such cells can be administered to a subject either as a population having a common HLA genotype, or as a mixed population of cells having different HLA genotypes. In either case, the data reported in Example 2 demonstrate that allogeneic administration of mixed populations of ASCs does not elicit an immune response in the recipient subject. One ramification of that finding recognized by the inventors is that ASCs from at least two donors may be administered to a subject.

As used herein the phrase "adherent cells" refers to a homogeneous or heterogeneous population of cells which are anchorage dependent *in vitro, i.e*., which require attachment to a surface or to other cells in order to grow *in vitro.*

As used herein the term "placenta" refers to any portion of the mammalian female organ which lines the uterine wall and during pregnancy envelopes the fetus, to which it is attached by the umbilical cord. Following birth, the placenta is expelled (and is referred to as a post partum placenta). In some embodiments, "placenta" refers to whole placenta.

Placenta derived adherent cells may be obtained from both fetal (i.e., amnion or inner parts of the placenta, see Example 1) and maternal (i.e., decidua basalis, and decidua parietalis) parts of the placenta. In general, tissue specimens are washed in a physiological buffer [e.g., phosphate-buffered saline (PBS) or Hank's buffer] and single-cell suspensions are made by treating the tissue with a digestive enzyme or a mixture of digestive enzymes (see below) or/and mincing and flushing the tissue parts through a nylon filter or by gentle pipetting with washing medium.

Placenta derived adherent cells can be propagated using two dimensional or three dimensional culturing conditions. Nonlimiting examples of such culture conditions are provided in Example 1.

As used herein the phrase "three dimensional culture" refers to a culture in which the cells are exposed to conditions which are compatible with cell growth while allowing the cells to grow in more than one layer. It is well appreciated that the *in situ* environment of a cell in a living organism (or a tissue) is in a three dimensional architecture. Cells are surrounded by other cells. They are held in a complex network of extra cellular matrix nanoscale fibers that allows the establishment of various local microenvironments. Their extra cellular ligands mediate not only the attachment to the basal membrane but also access to a variety of vascular and lymphatic vessels. Oxygen, hormones and nutrients are ferried to cells and waste products are carried away. The conditions in the three dimensional culture are designed to mimic certain aspects of such an environment as is further exemplified below. It will be appreciated that the conditions of the three-dimensional culture are such that enable expansion of the adherent cells.

As used herein the terms "expanding" and "expansion" refer to substantially differentiation-less maintenance of the cells and ultimately cell growth, *i.e*., increase of a cell population (*e.g.,* at least 2 fold) without terminal differentiation accompanying such increase.

Examples of adherent materials which may be used to culture cells as described herein include, but are not limited to, a polyester, a polypropylene, a polyalkylene, a polyfluorochloroethylene, a polyvinyl chloride, a polystyrene, a polysulfone, a cellulose acetate, a glass fiber, a ceramic particle, a matrigel, an extra cellular matrix component (e.g., fibronectin, chondronectin, laminin), a collagen, a poly L lactic acid and an inert metal fiber.

Non-limiting examples of base media useful in culturing placental derived cells to derive ASCs include Minimum Essential Medium Eagle, ADC-1, LPM (Bovine Serum Albumin-free), F10(HAM), F12 (HAM), DCCM1, DCCM2, RPMI 1640, BGJ Medium (with and without Fitton-Jackson Modification), Basal Medium Eagle (BME-with the addition of Earle's salt base), Dulbecco's Modified Eagle Medium (DMEM-without serum), Yamane, IMEM-20, Glasgow Modification Eagle Medium (GMEM), Leibovitz L-15 Medium, McCoy's 5A Medium, Medium M199 (M199E-with Earle's sale base), Medium M199 (M199H-with Hank's salt base), Minimum Essential Medium Eagle (MEM-E-with Earle's salt base), Minimum Essential Medium Eagle (MEM-H-with Hank's salt base) and Minimum Essential Medium Eagle (MEM-NAA with non essential amino acids), among numerous others, including medium 199, CMRL 1415, CMRL 1969, CMRL 1066, NCTC 135, MB 75261, MAB 8713, DM 145, Williams' G, Neuman & Tytell, Higuchi, MCDB 301, MCDB 202, MCDB 501, MCDB 401, MCDB 411, MDBC 153. In some embodiments the medium is DMEM. These and other useful media are available from GIBCO, Grand Island, N.Y., USA and Biological Industries, Bet HaEmek, Israel, among others. A number of these media are summarized in Methods in Enzymology, Volume LVIII, "Cell Culture", pp. 62 72, edited by William B. Jakoby and Ira H. Pastan, published by Academic Press, Inc.

The medium may be supplemented such as with serum such as fetal serum of bovine or other species, and optionally or alternatively, growth factors, vitamins (e.g. ascorbic acid), cytokines, salts (*e.g.* B-glycerophosphate), steroids (e.g. dexamethasone) and hormones e.g., growth hormone, erythropoeitin, thrombopoietin, interleukin 3, interleukin 6, interleukin 7, macrophage colony stimulating factor, c-kit ligand/stem cell factor, osteoprotegerin ligand, insulin, insulin like growth factors, epidermal growth factor, fibroblast growth factor, nerve growth factor, cilary neurotrophic factor, platelet derived growth factor, and bone morphogenetic protein at concentrations of between picogram/ml to milligram/ml levels.

The skilled artisan will appreciate that additional components may be added to the culture medium. Such components may be antibiotics, antimycotics, albumin, amino acids, and other components known to the art for the culture of cells. Additionally, components may be added to enhance the differentiation process when desirable.

As mentioned, once adherent cells are at hand they may be passaged to two dimensional or three dimensional settings (see Example 1). It will be appreciated though, that alternative embodiments are also possible in which the cells are transferred to a 3D-configured matrix immediately after isolation or alternatively, may be passaged to three dimensional settings following two dimensional conditions.

Thus, the adherent material is configured for 3D culturing thereby providing a growth matrix that substantially increases the available attachment surface for the adherence of the cells so as to mimic the infrastructure of the tissue (*e.g.,* placenta).

Examples of 3D bioreactors include, but are not limited to, a plug flow bioreactor, a continuous stirred tank bioreactor, a stationary-bed bioreactor, a CelliGen Plus^{®} bioreactor system (New Brunswick Scientific (NBS) or a BIOFLO 310 bioreactor system (New Brunswick Scientific (NBS).

As shown Example 1, the Celligen bioreactor is capable of 3D expansion of adherent cells under controlled conditions (*e.g.,* pH, temperature and oxygen levels) and with constant cell growth medium perfusion. Furthermore, the cell cultures can be directly monitored for concentration levels of glucose, lactate, glutamine, glutamate and ammonium. The glucose consumption rate and the lactate formation rate of the adherent cells can be used to measure cell growth rate and to determine the harvest time.

Other 3D bioreactors that can be used include, but are not limited to, a continuous stirred tank bioreactor [where a culture medium is continuously fed into the bioreactor and a product is continuously drawn out to maintain a time-constant steady state within the reactor], a stirred tank bioreactor with a fibrous bed basket [available, for example, from New Brunswick Scientific Co., Edison, NJ], a stationary-bed bioreactor, an air-lift bioreactor [where air is typically fed into the bottom of a central draught tube flowing up while forming bubbles, and disengaging exhaust gas at the top of the column], a cell seeding perfusion bioreactor with Polyactive foams [as described in Wendt, D. et al., Biotechnol Bioeng 84: 205-214, (2003)], and tubular poly-L-lactic acid (PLLA) porous scaffolds in a Radial-flow perfusion bioreactor [as described in Kitagawa et al., Biotechnology and Bioengineering 93(5): 947-954 (2006)]. Other bioreactors which can be used are described in U.S. Pat. Nos. 6,277,151, 6,197,575, 6,139,578, 6,132,463, 5,902,741 and 5,629,186.

Cell seeding is preferably effected at a concentration of 100,000-1,500,000 cells / ml at seeding. In an exemplary embodiment a total of 150 ± 30 x 10⁶ cells are seeded, 3-5 x 10⁶ cell / g carrier are seeded, or 0.015-0.1 x 10⁶ cell / ml are seeded.

In some embodiments the ASCs are positive for at least one marker selected from CD73, CD90, CD29, D7-FIB and CD105. A population is positive for a marker if the population contains a proportion of cells positive for the marker such that expression of the marker above a threshold level can be detected in the population as a whole. Threshold levels can be determined, for example, by comparison to a known negative population of cells, by omission of a reagent used in the detection protocol, or by substitution of a non-detecting reagent, such as an isotype control, in the detection protocol. In some embodiments expression is measured on a cell by cell basis, such as using a FACS analysis, while in others it is measured on an entire sample of the population at once, such as using a Western Blot. In some embodiments positive expression of the marker in the population is defined as detectable expression by at least 5%, at least 10%, at least 20%, or at least 50% or more of the cells in the population.

In some embodiments the ASCs are negative for at least one marker selected from CD3, CD4, CD45, CD80, HLA-DR, CD11b, CD14, CD19, CD34, CD200 and CD79. A population is negative for a marker if the population contains so few cells positive for the marker that expression of the marker above a threshold level can not be detected in the population as a whole. Threshold levels can be determined, for example, by comparison to a known negative population of cells, by omission of a reagent used in the detection protocol, or by substitution of a non-detecting reagent, such as an isotype control, in the detection protocol. In some embodiments expression is measured on a cell by cell basis, such as using a FACS analysis, while in others it is measured on an entire sample of the population at once, such as using a Western Blot. In some embodiments negative expression of the marker in the population is defined as detectable expression by less than 50%, less than 20%, less than 10%, and less than 5% of the cells in the population.

A pharmaceutical composition comprising ACSs from at least two donor placentas can be formed by mixing placental-derived cells at any point following harvesting of a placenta. By way of non-limiting example, the pharmaceutical composition can be made by a method comprising at least one step selected from mixing placental-derived cells prior to culturing *in vitro,* mixing placental-derived cells during 2D culturing, mixing placental-derived cells after 2D culturing, mixing placental-derived cells during 3D culturing, and mixing placental-derived cells after 3D culturing. The compositions comprising the ASCs may be subdivided and stored as aliquots comprising at least one effective amount of the ASCs. The aliquots may be prepared in tubes, bags, or any other container suitable for preserving the at least one effective amount of ASCs for use in the methods described.

In some embodimients, the ASCs are capable of suppressing immune reaction in a subject. As used herein the phrase "suppressing immune reaction in a subject" refers to decreasing or inhibiting the immune reaction occurring in a subject in response to an antigen (e.g., a foreign cell or a portion thereof). The immune response which can be suppressed by the adherent cells include the humoral immune responses, and cellular immune responses, which involve specific recognition of pathogen antigens via antibodies and T-lymphocytes (proliferation of T cells), respectively.

As used herein the term "treating" refers to inhibiting or arresting the development of a disease or condition (*e.g.,* ischemia) and/or causing the reduction, remission, or regression of the disease or condition. In some embodiments the inhibition or arrest is accompanied by the reduction, remission, or regression or at least one symptom of the disease or condition. Those of skill in the art will understand that various methodologies and assays can be used to assess the development of a disease or condition, and similarly, various methodologies and assays may be used to assess the reduction, remission or regression of a disease or condition.

The phrase "connective tissue" refers to a supporting framework tissue comprising strands of collagen, elastic fibers (e.g., between and around muscle and blood vessels) and simple cells. Examples of connective tissues include, but are not limited to dense connective tissue (e.g., ligament, tendon, periodontal ligament), areolar connective tissue (e.g., with proteinaceous fibers such as collagen and elastin), reticular connective tissue, adipose tissue, blood, bone, cartilage, skin, intervertebral disc, dental pulp, dentin, gingival, extracellular matrix (ECM)-forming cells, loose connective tissue and smooth muscle cells.

The term "ischemia" as used herein refers to any pathology (disease, condition, syndrome or disorder) characterized by or associated with insufficient angiogenesis. Examples include, but are not limited to, a peripheral arterial disease (PAD) such as limb ischemia and critical limb ischemia (CLI), ischemic heart disease, ischemic brain disease (e.g. stroke), delayed wound-healing, delayed ulcer healing, reproduction associated disorders, arteriosclerosis, ischemic vascular disease, ischemic heart disease, myocardial ischemia, coronary artery disease (CAD), atherosclerotic cardiovascular disease, left main coronary artery disease, arterial occlusive disease, peripheral ischemia, peripheral vascular disease, vascular disease of the kidney, peripheral arterial disease, limb ischemia, lower extremity ischemia, cerebral ischemia, cerebro vascular disease, retinopathy, retinal repair, remodeling disorder, von Hippel-Lindau syndrome, hereditary hemorrhagic telengiectasiaischemic vascular disease, Buerger's disease, ischemic renal disease and ischemic placenta.

As used herein the phrase "medical condition requiring connective tissue regeneration and/or repair" refers to any pathology characterized by connective tissue damage (i.e., non-functioning tissue, cancerous or pre-cancerous tissue, broken tissue, fractured tissue, fibrotic tissue, or ischemic tissue) or loss (e.g., following a trauma, an infectious disease, a genetic disease, and the like). Non-limiting examples of such pathologies include, bone fracture, bone cancer (e.g., osteosarcoma, bone cancer metastasis), burn wound, articular cartilage defect and deep wound.

Since non-autologous cells may induce an immune reaction when administered to the body several approaches have been developed to reduce the likelihood of rejection of non-autologous cells. These include, for example, either suppressing the recipient immune system or encapsulating the non-autologous cells in immunoisolating, semipermeable membranes before transplantation.

Encapsulation techniques are generally classified as microencapsulation, involving small spherical vehicles and macroencapsulation, involving larger flat-sheet and hollow-fiber membranes (Uludag, H. et al. Technology of mammalian cell encapsulation. Adv Drug Deliv Rev. 2000; 42: 29-64).

Methods of preparing microcapsules are known in the arts and include for example those disclosed by Lu MZ, et al., Cell encapsulation with alginate and alpha-phenoxycinnamylidene-acetylated poly(allylamine). Biotechnol Bioeng. 2000, 70: 479-83, Chang TM and Prakash S. Procedures for microencapsulation of enzymes, cells and genetically engineered microorganisms. Mol Biotechnol. 2001, 17: 249-60, and Lu MZ, et al., A novel cell encapsulation method using photosensitive poly(allylamine alpha-cyanocinnamylideneacetate). J Microencapsul. 2000, 17: 245-51.

For example, microcapsules are prepared by complexing modified collagen with a ter-polymer shell of 2-hydroxyethyl methylacrylate (HEMA), methacrylic acid (MAA) and methyl methacrylate (MMA), resulting in a capsule thickness of 2-5 µm. Such microcapsules can be further encapsulated with additional 2-5 µm ter-polymer shells in order to impart a negatively charged smooth surface and to minimize plasma protein absorption (Chia, S.M. et al. Multi-layered microcapsules for cell encapsulation Biomaterials. 2002 23: 849-56).

Other microcapsules are based on alginate, a marine polysaccharide (Sambanis, A. Encapsulated islets in diabetes treatment. Diabetes Technol. Ther. 2003, 5: 665-8) or its derivatives. For example, microcapsules can be prepared by the polyelectrolyte complexation between the polyanions sodium alginate and sodium cellulose sulphate with the polycation poly(methylene-co-guanidine) hydrochloride in the presence of calcium chloride.

It will be appreciated that cell encapsulation is improved when smaller capsules are used. Thus, the quality control, mechanical stability, diffusion properties, and *in vitro* activities of encapsulated cells improved when the capsule size was reduced from 1 mm to 400 µm (Canaple L. et al., Improving cell encapsulation through size control. J Biomater Sci Polym Ed. 2002;13:783-96). Moreover, nanoporous biocapsules with well-controlled pore size as small as 7 nm, tailored surface chemistries and precise microarchitectures were found to successfully immunoisolate microenvironments for cells (Williams D. Small is beautiful: microparticle and nanoparticle technology in medical devices. Med Device Technol. 1999, 10: 6-9; Desai, T.A. Microfabrication technology for pancreatic cell encapsulation. Expert Opin Biol Ther. 2002, 2: 633-46).

Examples of immunosuppressive agents include, but are not limited to, methotrexate, cyclophosphamide, cyclosporine, cyclosporin A, chloroquine, hydroxychloroquine, sulfasalazine (sulphasalazopyrine), gold salts, D-penicillamine, leflunomide, azathioprine, anakinra, infliximab (REMICADE), etanercept, TNFablockers, a biological agent that targets an inflammatory cytokine, and Non-Steroidal Anti-Inflammatory Drug (NSAIDs). Examples of NSAIDs include, but are not limited to acetyl salicylic acid, choline magnesium salicylate, diflunisal, magnesium salicylate, salsalate, sodium salicylate, diclofenac, etodolac, fenoprofen, flurbiprofen, indomethacin, ketoprofen, ketorolac, meclofenamate, naproxen, nabumetone, phenylbutazone, piroxicam, sulindac, tolmetin, acetaminophen, ibuprofen, Cox-2 inhibitors and tramadol.

### Conditions and Diseases That Can Be Treated With ASCs

Peripheral arterial disease (PAD) is a chronic disease that progressively restricts blood flow in the limbs that can lead to serious medical complications. This disease is often associated with other clinical conditions, including hypertension, cardiovascular disease, hyperlipidemia, diabetes, obesity and stroke. Critical Limb Ischemia (CLI) is used to describe patients with chronic ischemia induced pain, ulcers, tissue loss or gangrene in the limb. CLI represents the end stage of PAD patients who need comprehensive treatment by a vascular surgery or vascular specialist. In contrast to coronary and cerebral artery disease, peripheral arterial disease (PAD) remains an under-appreciated condition that despite being serious and extremely prevalent is rarely diagnosed and even less frequently treated. Consequently, CLI often leads to amputation or death and mortality rates in PAD patients exceed that of patients with myocardial infarction and stroke.

In attempts to treat ischemic conditions, various adult stem cells have been used. Thus, co-culturing of adipose tissue derived stromal cells (ADSC) and endothelial cells (EC) resulted in a significant increase in EC viability, migration and tube formation mainly through secretion of VEGF and HGF. Four weeks after transplantation of the stromal cells into the ischemic mouse hind limb the angiogenic scores were improved [Nakagami et al., J Atheroscler Thromb (2006) 13(2): 77-81]. Moon et al. [Cell Physiol Biochem. (2006) 17: 279-90] have tested the ability of ADSC to treat limb ischemia in immunodeficient mice and demonstrated a significant increase in the laser Doppler perfusion index in ADSC-transplanted group.

In addition, when umbilical cord blood (UCB)-derived mesenchymal stem cells were transplanted into four men with Buerger's disease who had already received medical treatment and surgical therapies, ischemic rest pain, suddenly disappeared from their affected extremities [Kim et al., Stem Cells (2006) 24(6): 1620-6]. Moreover, transplantation of human mesenchymal stem cells isolated from fetal membranes of term placenta (FMhMSC) into infarcted rat hearts was associated with increased capillary density, normalization of left ventricular function, and significant decrease in scar tissue, which was enhanced when the stem cells were preconditioned with a mixed ester of hyaluronan with butyric and retinoic acid [Ventura et al., (2007) J. Biol. Chem., 282: 14243-52].

Stroke is one of the leading causes of death around the world. Although there has been a constant reduction in stroke mortality in developed countries, probably due to improved control of stroke risk factors (especially high blood pressure, diabetes and cigarette smoking), stroke still leads to permanent damage (*e.g.* tissue damage, neurological damage).

New treatment regimens for stroke include stem cell therapy. Transplantation of stem cells or progenitors into the injured site, either locally or via intravenous routes, to replace nonfunctional cells, enhance proliferation and/or differentiation of endogenous stem or progenitor cells and supply necessary immune modulators has been contemplated and stand as the major cell-based strategy. Potential sources of stem/progenitor cells for stroke include fetal neural stem cells, embryonic stem cells, neuroteratocarcinoma cells, umbilical cord blood-derived non-hematopoietic stem cells, bone marrow-derived stem cells and placental-derived mesenchymal stem cells [Andres et al., Neurosurg Focus (2008) 24(3-4): E16].

In a recent study, Koh et. al. [Koh et al., Brain Res. (2008)] examined the neuroprotective effects and mechanisms of implanted human umbilical cord-derived mesenchymal stem cells (hUC-MSCs) in an ischemic stroke rat model. Twenty days after the induction of in-vitro neuronal differentiation, hUC-MSCs displayed morphological features of neurons and expressed neuronal cell markers and neuronal factors (e.g. glial cell line-derived neurotrophic factor, brain-derived neurotrophic factor). Furthermore, in-vivo implantation of the hUC-MSCs into the damaged hemisphere of immunosuppressed ischemic stroke rats improved neurobehavioral function and reduced infarct volume relative to control rats. Three weeks after implantation, hUC-MSCs were present in the damaged hemisphere and expressed neuron-specific markers, yet these cells did not become functionally active neuronal cells.

Various conditions and pathologies require connective tissue (e.g., bone, tendon and ligament) regeneration and/or repair. These include, for example, bone fractures, burns, burn wound, deep wound, degenerated bone, various cancers associated with connective tissue loss (e.g., bone cancer, osteosarcoma, bone metastases), and articular cartilage defect.

The use of autologous BM-MSCs to enhance bone healing has been described for veterinary and human orthopedic applications and include percutaneous injection of bone marrow for ligament healing (Carstanjen et al., 2006), treatment of bone defects by autografts or allografts of bone marrow in orthopedic clinic (Horwitz et al., 1999, Horwitz et al., 2002), regeneration of critical-sized bone defect in dogs using allogeneic [Arinzeh TL, et al., J Bone Joint Surg Am. 2003, 85-A(10):1927-35] or autologous [Bruder SP, et al., J Bone Joint Surg Am. 1998 Jul;80(7):985-96] bone marrow-MSCs loaded onto ceramic cylinder consisting of hydroxyapatite-tricalcium phosphate, or in rabbit using allogeneic peripheral blood derived MSCs (Chao et al., 2006.), and extensive bone formation using MSCs implantation in baboon (Livingston et al, 2003).

Within the equine orthopedic field, mesenchymal stem cells of BM and adipose sources have been used experimentally for surgical treatment of subchondral- bone cysts, bone fracture repair [Kraus and Kirker-Head,Vet Surg (2006) 35(3): 232-42] and cartilage repair [Brehm et al., Osteoarthritis Cartilage (2006) 14(12): 1214-26; Wilke et al., J Orthop Res (2007) 25(7): 913-25] and clinically in the treatment of overstrain induced injuries of tendons in horses. Furthermore, different therapeutic approaches have been used to promote suspensory ligament healing in horses (Herthel, 2001). Herthel (2001) have demonstrated a novel biological approach to facilitate suspensory ligament healing that involves the intra lesional injection of autologous stem cells and associated bone marrow components to stimulate natural ligament regeneration.

Rabbit models for injured tendons showed that MSC-treated tissues were stronger and stiffer than natural repaired tissues (Gordon et al., 2005). In addition, seeding of cultured MSCs into a tendon gap resulted in significantly improved repair biomechanics (Young et al., 1998, Osiris Therapeutics, www.osiris.com). Osiris Chondrogen (adult Mesenchymal Stem Cells) is being tested in patients in order to evaluate safety and efficacy. In MSC treated animals, surgically removed meniscal tissue was regenerated, the cartilage surface was protected, and lessened joint damage was observed in comparison to control animals. These benefits persisted in animal models at least through one year (Osiris Therapeutics, www.osiris.com).

Inflammatory bowel disease (IBD), a group of inflammatory conditions of the large intestine and small intestine, includes Crohn's disease and ulcerative colitis and is a chronic, relapsing, and remitting condition of an unknown origin. Crohn's disease (also known as granulomatous colitis and regional enteritis), an autoimmune disease caused by the immune system's attacking the gastrointestinal tract and producing inflammation in the gastrointestinal tract, is an inflammatory disease that may affect any part of the gastrointestinal tract from mouth to anus, causing a wide variety of symptoms. It primarily causes abdominal pain, diarrhea, vomiting and weight loss, but may also cause complications outside of the gastrointestinal tract such as skin rashes, arthritis and inflammation of the eye. There is currently no known drug or surgical cure for Crohn's disease and treatment options are restricted to controlling symptoms, maintaining remission and preventing relapse (using, *e.g.,* 5-aminosalicylic acid (5-ASA) formulations, corticosteroids such as prednisone and hydrocortisone, immunomodulators such as azathioprine and mercaptopurine, and biologic anti-tumor necrosis factor alpha agents). Ulcerative colitis, a form of colitis, is a disease of the intestine, specifically the large intestine or colon that includes characteristic ulcers, or open sores, in the colon. The main symptom of active disease is usually constant diarrhea mixed with blood. Current treatment of ulcerative colitis is similar to Crohn's disease. Colectomy (partial or total removal of the large bowel through surgery) is occasionally necessary. The use of ASCs in the treatment of inflammatory diseases of the colon is described in WO2009/144720, published 3 December 2009.

### EXAMPLES

### Example 1: Manufacture of 3D Adherent Cells

In order to provide large scale 3D adherent cells, a new manufacturing system was utilized referred to herein as Celligen.

### Materials and Experimental Methods

Celligen™ Plug Flow bioreactor-The production of adherent cells by CelligenTM (PLX-C cells) is composed of several major steps as illustrated in Figure 1A. The process starts by collection of a placenta from a planned cesarean delivery at term.

Adherent cells are then isolated from whole placentas, grown in tissue culture flasks (2D cultures), harvested and stored in liquid nitrogen as 2D-Cell Stock (2DCS), the appropriate amount of 2DCS are thawed and seeded onto carriers in bioreactors for further expansion as 3D-culture. After 4-14 days of growth in the bioreactors, cells are harvested and cryopreserved in gas phase of liquid nitrogen as PLX-C.

All placentas obtained were received from the maternity ward under approval of the Helsinki Committee of the medical facility. Accordingly, all placenta donors signed an informed consent and Donor Screening and Donor Testing was performed. Immediately after taking the placenta from the donor (during the caesarean procedure), it was placed in a sterile plastic bag and then in a Styrofoam box with ice packs. The placenta was delivered and immediately placed in a quarantine area until released to use by Quality Control (QC) and Quality Assurance (QA). All the following production steps were performed in a quarantine, clean room facility until QC approval of mycoplasma test results arrived and the cells were release for 2D cell growth.

To initiate the process, the whole placenta was cut into pieces under aseptic conditions under laminar flow hood, washed with Hank's buffer solution and incubated for 3 hours at 37 °C with 0.1 % Collagenase (1 mg Collagenase/ml tissue). 2D cell medium (2D-Medium comprising DMEM supplemented with 10 % FBS, fungizone 0.25 µg/ml and gentamycine 50 µg/ml) was added and the digested tissue was roughly filtered through a sterile metal strainer, collected in a sterile beaker and centrifuged (10 minutes, 1200 RPM, 4 °C). Using gentle pipeting, suspended cells were then washed with 2D-Medium supplemented with antibiotics, seeded in 80 cm2 flasks and incubated at 37 °C in a tissue culture incubator under humidified condition supplemented with 5 % CO2. Following 2-3 days, in which the cells were allowed to adhere to the flask surface, they were washed with PBS and 2D-Medium was added.

Two Dimensional (2D) Cell Growth - Prior to the first passage, growth medium samples of 10 % of the total flask number in quarantine was pooled and taken for mycoplasma testing. If cells were found to be negative for Mycoplasma (EZ-PCR Mycoplasma kit, Biological Industries, Israel), cells were released from quarantine. After 1-2 additional passages, cells were transferred to the 2D production clean room (2DP). Once in Room 2DP, culture was continued for another 3-5 passages. Sample was taken for immune phenotype after passage 4. Throughout the process, cultures were grown in 2D-Medium without antibiotics in a tissue culture incubator under humidified conditions with 5 % CO2 at 37 °C. After a total of 6-8 passages (9-16 cell doublings), cells were collected and cryopreserved as the 2D-Cell Stock (2DCS).

The first passage was usually carried out after 10-15 days. Beginning at passage 2 and continuing until passage 6-8, cells were passaged when the culture reached 70-80 % confluence, usually after 3-5 days (1.5-2 doublings). The cells were detached from the flasks using 0.25 % trypsin-EDTA (4 minutes at 37 °C) and seeded in a culture density of 3 ± 0.2 x 10³ cells/cm2. The size of the tissue culture flasks raised as the passages proceed. The culturing process started in 80 cm2 tissue culture flask, continued in 175 cm2, then in 500 cm2 (Triple flask) and finally the cells were seeded into Cell Factory 10 tray (6320 cm2).

Cryopreservation Procedure for 2D-Cell-Stock Product - For 2DCS cryopreservation, 2D-cultured cells were collected under aseptic conditions using 0.25 % trypsin-EDTA. The cells were centrifuged (1200 RPM, 10', 4 °C), counted and re-suspended in 2D-Medium.

For freezing, cell suspensions were diluted 1:1 with 2D-Freezing Mixture (final concentrations was 10 % DMSO, 40 % FBS and 50 % 2D-Medium). Approximately 1.5 - 2.5 x 10⁹ cells were manufactured from one placenta. 4 ml of the cells were stored at a final concentration of 10 x 10⁶/ ml in 5ml cryopreservation polypropylene vials. The vials were labeled and transferred to a controlled rate freezer for a graduated temperature reducing process (1 °C/min), after which they were transferred to storage in gas-phase of a liquid nitrogen freezer located in the Cold Storage Room. This material was referred to as the 2D-Cell Stock (2DCS) batch.

Initiation of the Three Dimensional (3D) Culture Procedures - To begin 3D culture, an appropriate amount (150 ± 30 x 10⁶) of cells from 2DCS were thawed in the 2DP room and washed with 3D-Medium (DMEM with 10 % FBS and 20 Mm Hepes) to remove DMSO prior to seeding in the prepared-in-advanced bioreactor systems. The content of each 2DCS vial was pipetted and diluted 1:9 with pre-warmed (37 °C) 3D-Medium. The cells were centrifuged (1200 RPM, 10', 4 °C) and re-suspended again in 50-100 ml pre-warmed (37 °C) 3D-Medium in a 250 ml sterile bottle. A sample was taken and cells were counted using a Trypan Blue stain in order to determine cell number and viability. The cell suspension was transferred under a laminar flow hood into a 0.5 L seeding bottle. From the seeding bottle the cell suspension was transferred via sterile tubing to the bioreactor by gravitation.

Production of 3D-adherent cells in the Celligen Bioreactor (PLX-C) - 3D growth phase was performed using an automatic CelliGen Plus® or BIOFLO 310 bioreactor system [(New Brunswick Scientific (NBS)] depicted in Figure 1B. The bioreactor system was used for cultivation of cells, in which conditions were suitable for high cell concentrations. The cultivation process was carried out using a bioreactor in a perfusion mode. The lab scale bioreactor was constructed of two main systems - the control system and the bioreactor itself (vessel and accessories). The parameters of the process were monitored and controlled by a control console which included connectors for probes, motor and pumps, control loops for Dissolved Oxygen (DO), pH, perfusion and agitation (with a motor), a gases control system, water circulation and heating system for temperature control and an operator interface. The controlled process parameters (such as temperature, pH, DO etc.) could be displayed on the operator interface and monitored by a designated controller.

As noted above, 150 ± 30 x 10⁶ cells from the cryopreserved 2DCS were thawed, washed and seeded in a sterile bioreactor. The bioreactor contained 30-50 gr carriers (FibraCel® disks, NBS), made of Polyester and Polypropylene and 1.5 ± 0.1 L 3D-Medium. The growth medium in the bioreactor was kept at the following conditions: 37 °C, 70 % Dissolved Oxygen (DO) and pH 7.3. Filtered gases (Air, CO2, N2 and 02) were supplied as determined by the control system in order to keep the DO value at 70 % and the pH value at 7.3. For the first 24 hours, the medium was agitated at 50 Rounds Per Minutes (RPM) and increased up to 200 RPM by day 2. For the first 2-3 days, the cells were grown in a batch mode. Perfusion was initiated when the medium glucose concentration decreased below 550 mg/liter. The medium was pumped from the feeding container to the bioreactor using sterile silicone tubing. All tubing connections were performed under laminar flow using sterile connectors. The perfusion was adjusted on a daily basis in order to keep the glucose concentration constant at approximately 550 ± 50 mg\liter. A sample of the growth medium was taken every 1-2 days for glucose, lactate, glutamine, glutamate and ammonium concentration determination (BioProfile 400 analyzer, Nova Biomedical). The glucose consumption rate and the lactate formation rate of the cell culture were used to measure cell growth rate. These parameters were then used to determine the harvest time based on accumulated experimental data.

The cell harvest process started at the end of the growth phase (4-10 days). Two samples of the growth medium were collected. One sample was prepared to be sent to an approved GLP laboratory for Mycoplasma testing according to USP and Eu standards, and the other one was transferred to a controlled rate freezer for a graduated temperature reducing process (1 °C/min), after which they were transferred to storage in gas-phase of a liquid nitrogen freezer located in the Cold Storage Room, in case a repeat Mycoplasma testing was needed. These medium samples were considered as part of the Mycoplasma testing of the final product and the results were considered as part of the criteria for product release.

The 3D-grown culture was harvested as follows:
The bioreactor vessel was emptied using gravitation via tubing to a waste container. The vessel was opened, by removing the head plate, and the carriers were aseptically transferred from the basket to the upper basket net (see Figure 1 B). The bioreactor vessel was then closed and refilled with 1.5 L pre-warmed PBS (37 °C). The agitation speed was increased to 150 RPM for 2 minutes. The PBS was drained and the washing procedure was repeated twice.

In order to release the cells from the carriers, 1.5 L pre-warmed to 37 °C Trypsin-EDTA (Trypsin 0.25 %, EDTA 1 mM) was added to the bioreactor vessel and carriers were agitated for 5 minutes in 150 RPM, 37 °C. Cell suspension was collected to a 5 L sterile container containing 250 ml FBS. Cell suspension was divided to 4 500 ml sterile centrifuge tubes and a Mycoplasma test sample was withdrawn. Cells were aseptically filled and cryopreserved as PLX-C.

FACS analysis of membrane markers - cells were stained with monoclonal antibodies as previously described. In short, 400,000-600,000 cells were suspended in 0.1 ml flow cytometer buffer in a 5 ml test tube and incubated for 15minutes at room temperature (RT), in the dark, with each of the following monoclonal antibodies (MAbs): FITC-conjugated anti-human CD29 MAb (eBioscience), PE conjugated anti human CD73 MAb (Becton Dickinson), PE conjugated anti human CD105 MAb (eBioscience), PE conjugated anti human CD90 MAb (Becton Dickinson), FITC-conjugated anti-human CD45 MAb (IQProducts), PE-conjugated anti-human CD19 MAb (IQProducts), PE conjugated anti human CD14 MAb (IQProducts), FITC conjugated anti human HLA-DR MAb (IQProduct), PE conjugated anti human CD34 MAb (IQProducts), FITC conjugated anti human CD31 MAb (eBioscience), FITC conjugated anti human KDR MAb (R&D systems), anti human fibroblasts marker (D7-FIB) MAb(ACRIS), FITC-conjugated anti-human CD80 MAb (BD), FITC-conjugated anti-human CD86 MAb (BD), FITC-conjugated anti-human CD40 MAb (BD), FITC-conjugated anti-human HLA-ABC MAb (BD), Isotype IgG1 FITC conjugated (IQ Products), Isotype IgG1 PE conjugated (IQ Products).

Cells were washed twice with flow cytometer buffer, resuspended in 500 µl flow cytometer buffer and analyzed by flow cytometry using FC-500 Flow Cytometer (Beckman Coulter). Negative controls were prepared with relevant isotype fluorescence molecules.

Mixed Lymphocyte Reaction (MLR) - 2 x 10⁵ peripheral blood (PB) derived MNC (from donor A) were stimulated with equal amount of irradiated (3000 Rad) PB derived MNCs (from donor B). Increasing amounts of PLX-Cs were added to the cultures. Three replicates of each group were seeded in 96-well plates. Cells were cultured in RPMI 1640 medium containing 20 % FBS. Plates were pulsed with 1 µC 3H-thymidine during the last 18 hrs of the 5-day culturing. Cells were harvested over a fiberglass filter and thymidine uptake was quantified with scintillation counter.

For CFSE staining, PB-MNC cells were stained for CFSE (Molecular Probes) for proliferation measurement before culturing. Cells were collected after 5 days and the intensity of CFSE staining was detected by Flow Cytometry.

### ELISA

ELISA was carried out as was previously described. In short, MNCs (isolated from peripheral blood) were stimulated with 5 µg/ml ConA (Sigma), 0.5 µg/ml LPS (SIGMA), or 10 µg/ml PHA (SIGMA) in the presence of PLX-C under humidified 5 % CO2 atmosphere at 37 °C. Supernatants were collected and subjected to cytokine analysis using ELISA kits for IFNγ (DIACLONE), TNFα (DIACLONE) and IL-10 (DIACLONE).

Expression of cellular markers on PLX-C cells - the surface antigens expressed by PLX-C were examined using monoclonal antibodies as described above. Results indicated that PLX-C cells were positive for the markers CD73, CD29 and CD105 and negative for the markers CD34, CD45, CD19, CD14 and HLA-DR (data not shown). The immune phenotype test specifications were set as: ≥ 90 % for all positive markers and ≤ 3 % for all negative markers.

Furthermore, as shown in Figures 2A-B, PLX-C cultures did not express endothelial markers as shown by negative staining for the two endothelial markers CD31 and KDR. However, PLX-C expression of a fibroblast-typical marker was evident (expression of D7-fib, Figure 2C).

Immunogenecity and immunomodulatory properties of PLX-C cells - As PLX-C is comprised of adherent cells derived from placenta, it is expected to express HLA type I, which is expressed by all cells of the body and is known to induce an alloreactive immune response. HLA type II and other co-stimulatory molecules are typically expressed only on the surface of Antigen Presenting Cells (APCs).

To examine the immunogenicity of the PLX-C cells, analysis of the expression of co-stimulatory molecules on the surface of these cells was performed. FACS analysis demonstrated the absence of detectable CD80, CD86 and CD40 on the PLX-C cell membranes (Figures 3A-C). Moreover, PLX-C expressed low levels HLA class I as detected by staining for HLA A/B/C (Figure 3D). The PLX-C were similar to bone marrow (BM) derived MSCs in their lack of expression of stimulatory and co-stimulatory molecules (as shown in Figures 3A-D).

To further investigate the immunogenecity as well as the immunomodulation properties of PLX-C cells, Mixed Lymphocyte Reaction (MLR) tests were performed. As shown in Figure 4A-B, PLX-C cells both escape allorecognition, and reduce T cell response, as measured by thymidine incorporation. Furthermore, the reduction in lymphocytes proliferation (evaluated by CPM measurement) was higher as the number of PLX-C cells increased (in a dose dependent manner). PLX-C also reduced lymphocyte proliferation following mitogenic stimuli, such as Concavalin A (Con A, Figure 4B) and Phytohemagglutinin (PHA), and non-specific stimulation by anti-CD3, anti-CD28 (data not shown).

In order to investigate the mechanism of action by which PLX-C immunomodulate lymphocyte proliferation, and to see if this action is mediated via cell to cell interaction or cytokines secretion, PB derived Mononuclear cells (MNCs) were stimulated by PHA using the transwell method (which prevents cell to cell contact but enables the diffusion of cytokines between the two compartments). Results showed that the inhibition of proliferation maintained even when cell to cell contact was inhibited (data not shown).

Cytokines secretion - as depicted hereinabove, PLX-C reduce the proliferation rate of lymphocytes, probably through soluble factors. Further investigation of the cytokines secreted by lymphocytes in response to PLX-C was performed to elucidate the mechanism of action of PLX-C. As depicted in Figures 5A-B, culturing of mononuclear cells with PLX-C slightly reduces the secretion of the pro-inflammatory cytokine INFγ and dramatically reduces the secretion of TNFα (even in the presence of low amounts of PLX-C). In addition, following lipopolysaccharide (LPS) stimulation, PB derived MNCs secretion of IL-10 increased in the presence of PLX-C, while the secretion level of TNFα decreased, in a dose dependent manner (Figure 5C).

### Example 2: No Significant T Cell Alloreactivity in Patients Treated with PLX Cells

In general, engraftment of cells that are unmatched in their histocompatibility antigens (HLA) to a recipient (*i.e*., allogeneic cells) will generate a robust host versus graft response leading to a rapid elimination of the cells from the recipient's bodv. The major immune cell driving this rejection response is the T cell. T cells can specifically identify foreign HLA bearing cells and destroy them.

ASCs derived from placenta have immunosuppressive characteristics and have been shown to exert therapeutic properties in various pre-clinical animal disease models despite their xenogeneic origin (*i.e*., engraftment between individuals of different species). ASCs prepared in accordance with Example 1 are thus being evaluated in a dose-escalating phase I clinical trial as an allogeneic product and, therefore, a concern is to ensure that patients do not develop an anti-ASC immune response.

Specifically, two phase I studies using ASCs, intended for treatment of critical limb ischemia (CLI), were designed to evaluate safety, including immunological profile associated with local administration. These open-label, dose-escalation studies were performed in parallel in the EU and U.S. The design of the studies is similar, but not identical. For example, the follow up period and dose escalation schedules differ following regulatory requirements and previous experience of the clinical sites. The clinical follow-up period for both studies is three months after treatment; however, in the EU, the patients are observed for 24 months, versus 12 months in the U.S.

Altogether five dosing groups were evaluated. For the low dose group in the U.S., ASCs were multiply injected during one course. For the high dose group the additional dosing was achieved by administering the cells by multiple injections during two courses, two weeks apart. In contrast, in the EU, the higher dose was administered in a single course of multiple injections, using higher volumes of cells per injection.

In the U.S., ASCs were administered via 30 intramuscular (IM) injections delivered to the affected leg for the low dose treatment group, while for the higher dose, ASCs was administered twice (two courses, two weeks apart), with 30 IM injections delivered to affected leg in each course. In the EU study, all three treatment groups were treated with 30 IM injections delivered to the affected leg.

In order to evaluate whether patients treated with ASCs had developed a specific T cell response to the ASCs, a series of blood tests was performed before and following cell injections. Peripheral blood mononuclear cells (PBMCs) from treated patients were subjected to an Enzyme Linked Immuno-Sorbent SPOT (ELISPOT) Assay in order to evaluate the frequency of their anti ASC T cell responses.

The ELISPOT assay is based on detecting interferon gamma secreting cells. Peripheral blood mononuclear cells (PBMCs) are separated from whole blood and incubated with the tested T cell antigen (e.g., peptide, protein or whole cells). The cells are then plated on a membrane that was precoated with interferon-specific antibodies. Specific T cells that respond to the tested component will then become stimulated and secrete interferon gamma protein. The anti interferon antibodies on the membrane will then capture interferon in proximity to the secreting cell. Next, the cells are washed away and the membranes are incubated with an enzyme conjugated anti interferon gamma antibody. After washing unbound antibodies, a substrate is added and transformed by the enzyme into a black precipitant on the membrane. Thus, each spot on the membrane represents an interferon gamma secreting cell. The number of spots per total PBMCs plated represents the frequency of T cells that are specific for a given antigen.

This assay was performed using a commercially available interferon ELISPOT kit (EliSpot Basiskit ELSP 5500, AID, Strassberg, GmbH) according to the manufacturer's protocol. Briefly, whole blood was drawn from patients at the day of injection (V2), and 24 hours (V3), and one week (V4) after ASC administration. PBMCs were separated from whole blood by Ficoll gradient centrifugation and stored at -80°C until tested. 300,000 cells were then stimulated with CMV IE-1, CMV pp65, EBV peptides and allogeneic cells (as positive control) and PLX cells or left un-stimulated as a negative control. Following stimulation, cells were incubated in interferon gamma ELISPOT well plates. Cells were then washed away and a biotinilated anti interferon gamma antibody was added to plates. After washing away residual antibody, a steptavidin conjugated alkaline phosphatase was added. The reagent was washed and the alkaline phosphatase substrate NBT/BCIP was then added to form a blue-black precipitant. Spots were counted and analyzed.

The following Table 1 shows the HLA genotypes of two batches (P110209 and P040509) of ASCs used in these experiments. As shown in the table, P110209 is a homogenous population as reflected in only two alleles at each locus. In contrast, P040509 is a mixed population having three alleles at the HLA-A and HLA-B loci. In this case the cells are derived from both maternal and fetal portions of the placenta.

**Table 1**

| Batch no. | Class I | | | | | | Class II | | | |
|---|---|---|---|---|---|---|---|---|---|---|
| | A | | | B | | | DR | | DQ | |
| P110209 | A*02 | A*11 | | B*35 | B*52 | | DRB1*0301 | DRB1*15 | DQB1*02 | DQB1*06 |
| P040509 | A*11 | A*24 | A*31 | B*35 | B*40 | B*51 | DRB1*11 | DRB1*15 | DQB1*03 | DQB1*06 |

In this assay, 300,000 PBMCs from PLX treated patients were stimulated *in vitro* with CMV and EBV peptides, PLX cells, allogeneic cells or left unstimulated. Cells were subjected to interferon gamma ELISPOT assay and number of spots per treatment were counted. The results are shown in the table 2.

**Table 2**

| | | | Antigen-specific T cell response | | | | | |
|---|---|---|---|---|---|---|---|---|
| patient symbol | batch number | visit number | CMV IE-1 | CMV pp65 | EBV | PLX-reactive | Background | PLX-Backgroud |
| hau 990 | P110209 R89 | V2 | 1 | 0 | 14 | 0 | 3 | -3 |
| | | V3 | 1 | 1 | 11 | 0 | 0 | 0 |
| | | V4 | 1 | 1 | 11 | 0 | 2 | -2 |
| zaq372 | P110209 R89 | V2 | 17 | TNTC * | 42 | 4 | 0 | 4 |
| | | V3 | 83 | TNTC | 193 | 7 | 7 | 0 |
| | | V4 | 43 | TNTC | 120 | 6 | 5 | 1 |
| eqo 406 | P040509 R12 | V2 | 13 | 164 | 7 | 1 | 4 | -3 |
| | | V3 | 10 | 136 | 7 | 6 | 3 | 3 |
| | | V4 | 0 | 3 | 0 | 1 | 0 | 1 |
| ooe 517 | P040509R34 | V2 | 2 | 45 | 0 | 0 | 0 | 0 |
| | | V3 | 1 | 0 | 0 | 0 | 0 | 0 |
| | | V4 | 0 | 0 | 0 | 0 | 0 | 0 |
| byi 136 | P040509R34 | V2 | 555 | 16 | 10 | 3 | 3 | 0 |
| | | V3 | 341 | 0 | 1 | 1 | 0 | 1 |
| | | V4 | 21 | 0 | 0 | 0 | 0 | 0 |
| icq 111 | P040509 R34 | V2 | n.d.** | n.d. | n.d. | 1 | 0 | 1 |
| | P040509 R78 | V3 | 15 | 70 | 5 | 2 | 0 | 2 |
| | | V4 | 2 | 10 | 0 | 0 | 0 | 0 |

| | | | | | | | | |
|---|---|---|---|---|---|---|---|---|
| *Not documented, **Spots are too dense to count | | | | | | | | |

The results in Table 2 show that most patients reacted to CMV or EBV peptides as expected because most of the adult population has been exposed to those viruses and the existence of anti CMV or EBV T cells is prevalent. In addition, the number of spots does not increase after treatment, indicating that immunomodulation characteristics of PLX cells have no systemic effect on anti CMV and EBV memory T cell response. Throughout the treatment the no or very low T cell reactivity to PLX cells is observed in either the homogenous or mixed cell populations. This result demonstrates for the first time that mixed populations of ASCs derived from human placenta can be administered to humans without eliciting an immune response. Thus, this result demonstrates that mixed populations of ASCs can be created from one or more placentas and administered together to human subjects for therapeutic indications.

### Example 3: Mixed populations of PLX-C Cells Also Inihibit Mitogen-Induced T Cell Proliferation

The phytohemagglutinin (PHA) test measures the rate by which placenta derived cells reduce the proliferation of lymphocytes following mitogenic PHA stimuli. The objective of this study was to evaluate the immunosupresive properties of placenta derived cells from a single donor in comparison to a cell population derived from multiple donors according to their ability to reduce the proliferation of PHA stimulated lymphocytes. The mixed HLA populations were either co-cultured *in vitro* prior to the PHA test or grown separately and mixed just prior PHA testing.

### Materials and Methods

a. Thaw the PLX vials (see Table 3) in RPMI medium.
b. After discarding the supernatant, suspend the pellet of each tube, add 3ml of RPMI medium to each tube and transport 0.3ml sample from each tube to a cedex cup (300µl) for CEDEX count).
c. Count the cells (the minimal required cells amount is 1.5*10⁶).
d. Transport to eppendorf tubes 0.4*10⁶ cells from each batch and fill up with RPMI to final volume of 1 ml.
e. Seed each batch at the 40,000cell/well concentration, 100µl/well in 96 wells plate in triplicates.
f. Perform x2 dilution of the 40,000cell/well concentration by adding 0.7 ml RPMI medium to the eppendorf tubes and seed each batch at the 20,000 cell/well concentration, 100µl/well in 96 wells plate in triplicates.
g. Thaw frozen PBMCs in RPMI medium.
h. Dislodge the acquired pellet and take a sample for direct count, diluted 1:10 in Turk's Buffer.
i. Count by hemacytometer.
j. Adjust PBMCs concentration to 2*10⁶ cells /ml using RPMI medium.
k. Fill the first 2 groups of wells (= 6 wells) of 96 wells plate with 100µl RPMI medium without cells (for PBMCs control groups - group A (negative) and group B (positive)).
l. Add 100µl of the PBMCs suspension to the first group of wells (200,000 PBMCs / well - group A, negative control).
m. Remove the required amount of PBMCs to a new 50ml tube and add 20µl PHA stock solution (1 mg PHA/ml in PBS) per each 1 ml of PBMCs suspension.
n. Culture PHA stimulated PBMCs in 96-well plate (except the first triplicate), 100µl suspension/ well.
o. Incubate the plate for 3 days in an incubator (370C, 5% CO2).
p. To quantify PBMCs proliferation use Click-iT EdU Cell Proliferation Assay (Invitrogen Cat.no.C35002)

### Results

Culturing PBMC in the presence of PHA results in proliferation of those cells. Figure 6 shows the PHA-induced proliferation rate of PBMC cultured in the presence of different numbers of PLX cells from three different donors (P300309R0506; P281209R1112; and P220609R0506) or a mixture of two donors (P281209R1112+P220609R0506). Figure 7 presents the average PBMC proliferation for PBMC cultured without addition of PHA, PBMC cultured with PHA but without addition of any PLX cells, and PBMC cultured in the presence of PHA and PLX cells from different donors. The percentage of proliferation using the proliferation of the PBMC+PHA as 100% is shown in Table 3.

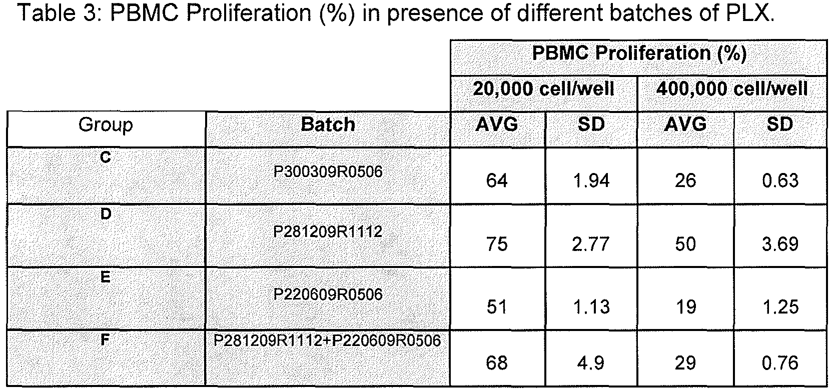

Mixing PLX cells from two donors resulted in an inhibition of PHA-stimulated proliferation that was intermediate to the effect observed when PLX cells for either donor were tested individually. Thus, PLX cells from different donors can be mixed and the PHA-induced inhibitory effect is still maintained.

## Claims

1. Placental-derived adherent stromal cells (ASCs) for use in therapy, wherein the ASCs comprise ASCs from at least two donor placentas.

2. Placental-derived adherent stromal cells (ASCs) for use for treating a condition that can be treated by administration of ASCs to a subject in need thereof, wherein the ASCs comprise ASCs from at least two donor placentas, and wherein the condition is selected from stem cell deficiency, heart disease, a neurodegenerative disorder, cancer, stroke, burns, loss of tissue, loss of blood, anemia, an autoimmune disease, ischemia, skeletal muscle regeneration, neuropathic pain, a compromised hematopoietic system, geriatric diseases, and a medical condition requiring connective tissue regeneration and/or repair.

3. The ASCs for the use of claims 1 or 2, wherein
(a) the ASCs are obtained by a method comprising culturing placental-derived cells in a three-dimensional (3D) culture; and/or
(b) wherein the ASCs are positive for at least one marker selected from CD73, CD90, CD29, D7-FIB and CD105.

4. The ASCs for the use of claim 3, wherein
(a) the 3D culturing comprises culturing in a 3D bioreactor;
(b) wherein the 3D culturing occurs for at least three days; and/or
(c) wherein the 3D culture step occurs until at least 10% of the cells are proliferating.

5. The ASCs for the use of claim 3(b), wherein the ASCs from each of the at least two donors are positive for the at least one marker.

6. The ASCs for the use of any one of claims 1 to 5, wherein
(a) the ASCs are negative for at least one marker selected from CD3, CD4, CD45, CD80, HLA-DR, CD11b, CD14, CD19, CD34, CD200, KDR, CD31 and CD79, optionally wherein the ASCs from each of the at least two donors are negative for the at least one marker;
(b) the ASCs are PLX or PLX-C cells;
(c) the neurodegenerative disorder is selected from multiple sclerosis (MS), Alzheimer's disease, and Parkinson's disease;
(d) the ischemia is peripheral arterial disease (PAD);
(e) the ischemia comprises ischemia of the central nervous system (CNS);
(f) the ischemia is selected from peripheral arterial disease, ischemic vascular disease, ischemic heart disease, ischemic brain disease, ischemic renal disease and ischemic placenta;
(g) the medical condition requiring connective tissue regeneration and repair is selected from bone fracture, bone cancer, burn wound, articular cartilage defect and deep wound;
(h) the medical condition requiring connective tissue regeneration and repair is selected from a subchondral-bone cyst, a bone fracture, an osteoporosis, an osteoarthritis, a degenerated bone, a cancer, a cartilage damage, an articular cartilage defect, a degenerative disc disease, an osteogenesis imperfecta (OI), a burn, a burn wound, a deep wound, a delayed wound-healing, an injured tendon and an injured ligament;
(i) the autoimmune disease is selected from rheumatoid arthritis, ankylosing spondylitis, inflammatory bowel disease (IBD), MS, diabetes type I, Goodpasture's syndrome, Graves' disease, Hashimoto's disease, Lupus, Myasthenia Gravis, Psoriasis, and Sjorgen's syndrome, wherein the IBD is optionally selected from Crohn's disease and ulcerative colitis;
(j) the administered ASCs comprise ASCs from at least three, at least four, at least five, at least ten, at least twenty-five, or at least 100 donors;
(k) the at least two donors have at least two different HLA genotypes, wherein the at least two different HLA genotypes are optionally genotypes of at least one of the HLA-A, HLA-B, HLA-DR, and HLA-DQ loci;
(l) the ASCs are administered to the subject in one treatment course, two treatment courses, not more than ten treatment courses, or in ten or more treatment courses;
(m) the ASCs are administered throughout the life of the subject;
(n) the ASCs from at least two donors are administered to the subject from at least one aliquot comprising ASCs from each of the at least two donors, wherein the aliquot comprising ASCs from each of the at least two donors is optionally made by a method comprising at least one step selected from mixing placental-derived cells prior to culturing in vitro, mixing placental-derived cells during 2D culturing, mixing placental-derived cells after 2D culturing, mixing placental-derived cells during 3D culturing, and mixing placental-derived cells after 3D culturing;
(o) the ASCs from at least two donors are administered to the subject from aliquots each comprising ASCs from only a single donor, wherein the administration of the ASCs from the aliquots from the at least two donors optionally occurs within 24 hours;
(p) the ASCs are administered for at least one treatment course, where the at least one treatment course comprises delivering from 1 to 40, from 5 to 40, from 10 to 30, about 5, about 10, about 15, about 20, about 25, about 30, about 35, about 40, or about 45, or about 50 separate injections to the subject, wherein optionally the subject is in need of treatment for critical limb ischemia and the ASCs are optionally administered to the subject by about 30 to about 50 intramuscular injections for one or two treatment courses; and/or
(q) the ASCs are obtained by a method comprising culturing placental-derived cells in a two-dimensional (2D) culture.

7. The ASCs for the use of claim 6(d), wherein the PAD is limb ischemia (LI).

8. The ASCs for the use of claim 7, wherein the limb ischemia is critical limb ischemia (CLI).

9. The ASCs for the use of any one of claims 1 to 5, wherein the connective tissue comprises at least one of tendon, bone and ligament.

10. The ASCs for the use of any one of claims 1 to 5, wherein the compromised hematopoietic system is caused by radiation or chemotherapy.

11. A pharmaceutical composition comprising adherent stromal cells (ASCs), wherein the pharmaceutical composition comprises ASCs from at least two donor placentas and a pharmaceutically acceptable carrier.

12. The pharmaceutical composition of claim 11, wherein
(a) the ASCs are obtained by a method comprising culturing placental-derived cells in a three-dimensional (3D) culture;
(b) the ASCs are positive for at least one marker selected from CD73, CD90, CD29, D7-FIB and CD105, wherein the ASCs from each of the at least two donors are optionally positive for the at least one marker;
(c) the ASCs are negative for at least one marker selected from CD3, CD4, CD45, CD80, HLA-DR, CD11b, CD14, CD19, CD34, CD200, KDR, CD31 and CD79, wherein the ASCs from each of the at least two donors are optionally negative for the at least one marker;
(d) the ASCs are PLX or PLX-C cells;
(e) the ASCs comprise ASCs from at least three, at least four, at least five, at least ten, at least twenty-five, or at least 100 donors;
(f) the at least two donors have at least two different HLA genotypes, wherein the at least two different HLA genotypes are optionally genotypes of at least one of the HLA-A, HLA-B, HLA-DR, and HLA-DQ Ioci;
(g) the ASCs are obtained by a method comprising culturing placental-derived cells in a two-dimensional (2D) culture; and/or
(h) the pharmaceutically acceptable carrier is an isotonic solution.

13. The pharmaceutical composition of claim 12(a), wherein the 3D culturing comprises culturing in a 3D bioreactor.

14. The ASCs for the use of claim 4, or the pharmaceutical composition of claim 13, wherein
(a) cells in the 3D bioreactor are cultured under perfusion; and/or
(b) the 3D bioreactor comprises at least one adherent material selected from a polyester and a polypropylene.

15. The pharmaceutical composition of claims 12(a), 13 or 14, wherein
(i) the 3D culturing occurs for at least three days; and/or
(ii) the 3D culture step occurs until at least 10% of the cells are proliferating.

16. The pharmaceutical composition of claim 12(h), wherein the isotonic solution further comprises about 5% human serum albumin, wherein the isotonic solution optionally further comprises about 5% to about 10% dimethyl sulphoxide.

17. The ASCs for the use of any one of claims 1-10, and 14 or the pharmaceutical composition of any one of claims 11-16, wherein the ASCs have three alleles at each of the HLA-A and HLA-B Ioci.

18. The ASCs for the use of any one of claims 1-10, and 14 or the pharmaceutical composition of any one of claims 11-16, wherein the ASCs are obtained from both maternal and fetal portions of the placenta.

19. The pharmaceutical composition of any one of claims 11-18, wherein the composition is suitable for administration by intravenous, intramuscular or subcutaneous injection.

## Patentansprüche

1. Adhärente Stromazellen (ASCs), die aus der Plazenta stammen, zur Verwendung in der Therapie, wobei die ASCs ASCs von mindestens zwei Spenderplazentas umfassen.

2. Adhärente Stromazellen (ASCs), die aus der Plazenta stammen, zur Verwendung bei der Behandlung eines Zustands, der durch Verabreichung von ASCs an ein bedürftiges Subjekt behandelt werden kann, wobei die ASCs ASCs von mindestens zwei Spenderplazentas umfassen und wobei der Zustand ausgewählt wird aus Stammzelldefizienz, Herzerkrankung, einer neurodegenerativen Störung, Krebs, Schlaganfall, Verbrennungen, Gewebsverlust, Blutverlust, Anämie, einer Autoimmunerkrankung, Ischämie, Skelettmuskelregeneration, neuropathischem Schmerz, einem beeinträchtigten hämatopoetischen System, Alterserkrankungen und einem medizinischen Zustand, der eine Regenration und/oder Reparatur von Bindegewebe erfordert.

3. ASCs zur Verwendung nach Anspruch 1 oder 2, wobei
(a) die ASCs durch ein Verfahren erhalten werden, welches das Kultivieren von Zellen, die aus der Plazenta stammen, in einer dreidimensionalen (3D) Kultur umfasst; und/oder
(b) wobei die ASCs positiv für mindestens einen Marker sind, der ausgewählt ist aus CD73, CD90, CD29, D7-FIB und CD105.

4. ASCs zur Verwendung nach Anspruch 3, wobei
(a) das 3D Kultivieren das Kultivieren in einem 3D Bioreaktor umfasst;
(b) wobei sich das 3D Kultivieren über mindestens drei Tage erstreckt; und/oder
(c) wobei der 3D Kulturschritt stattfindet, bis sich mindestens 10% der Zellen teilen.

5. ASCs zur Verwendung nach Anspruch 3(b), wobei die ASCs von jedem der mindestens zwei Spender positiv für den mindestens einen Marker sind.

6. ASCs zur Verwendung nach einem der Ansprüche 1 bis 5, wobei
(a) die ASCs negativ für mindestens einen Marker sind, der ausgewählt ist aus CD3, CD4, CD45, CD80, HLA-DR, CD11b, CD14, CD19, CD34, CD200, KDR, CD31 und CD79, optional wobei die ASCs von jedem der mindestens zwei Spender negativ für den mindestens einen Marker sind;
(b) die ASCs PLX oder PLX-C Zellen sind;
(c) die neurodegenerative Störung ausgewählt ist aus Multipler Sklerose (MS), Alzheimer Krankheit und Parkinsonscher Krankheit;
(d) die Ischämie eine Periphere Arterielle Krankheit (PAD) ist;
(e) die Ischämie eine Ischämie des zentralen Nervensystems (ZNS) umfasst;
(f) die Ischämie ausgewählt wird aus Peripherer Arterieller Krankheit, ischämischer Gefäßkrankheit, ischämischer Herzkrankheit, ischämischer Gehirnkrankheit, ischämischer Nierenkrankheit und ischämischer Plazenta;
(g) der medizinische Zustand, der eine Regeneration und Reparatur des Bindegewebes erfordert, ausgewählt ist aus Knochenfraktur, Knochenkrebs, Brandverletzung, Gelenkknorpeldefekt und tiefer Wunde.
(h) der medizinische Zustand, der eine Regeneration und Reparatur des Bindegewebes erfordert, ausgewählt ist aus einer subchondralen Knochenzyste, einer Knochenfraktur, einer Osteoporose, einer Osteoarthritis, einem degenerierten Knochen, einem Krebs, einem Knorpelschaden, einem Gelenkknorpeldefekt, einer degenerativen Bandscheibenerkrankung, einer Osteogenesis imperfecta (OI), einer Verbrennung, einer Brandwunde, einer tiefen Wunde, einer verzögerten Wundheilung, einer verletzten Sehne und einem verletzten Band;
(i) die Autoimmunerkrankung ausgewählt wird aus rheumatoider Arthritis, Spondylitis ankylosans, entzündlicher Darmerkrankung (IBD), MS, Diabetes typ I, Goodpasture-Syndrom, Morbus Basedow, Hashimoto-Thyreoiditis, Lupus, Myasthenia gravis, Psoriasis und Sjögren-Syndrom, wobei die IBD optional ausgewählt ist aus Morbus Crohn und Colitis ulcerosa;
(j) die verabreichten ASCs ASCs von mindestens drei, mindestens vier, mindestens fünf, mindestens zehn, mindestens fünfundzwanzig oder mindestens 100 Spendern umfassen;
(k) die mindestens zwei Spender mindestens zwei unterschiedliche HLA Genotypen aufweisen, wobei die mindestens zwei unterschiedlichen HLA Genotypen optional Genotypen mindestens eines der HLA-A, HLA-B, HLA-DR und HLA-DQ Loci sind;
(l) die ASCs dem Subjekt in einem Behandlungsgang, zwei Behandlungsgängen, nicht mehr als zehn Behandlungsgängen oder in zehn oder mehr Behandlungsgängen verabreicht werden;
(m) die ASCs über das gesamte Leben des Subjekts hinweg verabreicht werden;
(n) die ASCs von mindestens zwei Spendern dem Subjekt aus mindestens einem Aliquot verabreicht werden, das ASCs von jedem der mindestens zwei Spender umfasst, wobei das Aliquot, das ASCs von jedem der mindestens zwei Spender umfasst optional durch ein Verfahren hergestellt wird, das mindestens einen Schritt umfasst, der ausgewählt wird aus Mischen von Zellen, die aus der Plazenta stammen, vor dem Kultivieren in vitro, Mischen von Zellen, die aus der Plazenta stammen, während des 2D Kultivierens, Mischen von Zellen, die aus der Plazenta stammen, nach dem 2D Kultivieren, Mischen von Zellen, die aus der Plazenta stammen, während des 3D Kultivierens und Mischen von Zellen, die aus der Plazenta stammen, nach dem 3D Kultivieren;
(o) die ASCs von mindestens zwei Spendern dem Subjekt aus Aliquots verabreicht werden, die jeweils ASCs nur von einem einzelnen Spender umfassen, wobei die Verabreichung der ASCs aus den Aliquots von den mindestens zwei Spendern optional innerhalb von 24 Stunden stattfindet;
(p) die ASCs in mindestens einem Behandlungsgang verabreicht werden, wobei der mindestens eine Behandlungsgang das Verabreichen von 1 bis 40, von 5 bis 40, von 10 bis 30, etwa 5, etwa 10, etwa 15, etwa 20, etwa 25, etwa 30, etwa 35, etwa 40 oder etwa 45 oder etwa 50 separaten Injektionen an das Subjekt umfasst, wobei das Subjekt optional Bedarf an einer Behandlung von kritischer Ischämie der Extremitäten aufweist und die ASCs optional dem Subjekt durch etwa 30 bis etwa 50 intramuskuläre Injektionen in einem oder zwei Behandlungsgängen verabreicht werden; und/oder
(q) die ASCs durch ein Verfahren erhalten werden, welches das Kultivieren von Zellen, die aus der Plazenta stammen, in einer zweidimensionalen (2D) Kultur umfasst.

7. ASCs zur Verwendung nach Anspruch 6(d), wobei die PAD eine Ischämie der Gliedmaßen (LI) ist.

8. ASCs zur Verwendung nach Anspruch 7, wobei die Ischämie der Gliedmaßen eine kritische Ischämie der Gliedmaßen (CLI) ist.

9. ASCs zur Verwendung nach einem der Ansprüche 1 bis 5, wobei das Bindegewebe mindestens eine Sehne, einen Knochen oder ein Band umfasst.

10. ASCs zur Verwendung nach einem der Ansprüche 1 bis 5, wobei das beeinträchtigte hämatopoetische System durch Bestrahlung oder Chemotherapie verursacht wird.

11. Pharmazeutische Zusammensetzung, die adhärente Stromazellen (ASCs) umfasst, wobei die pharmazeutische Zusammensetzung ASCs von mindestens zwei Spenderplazentas und einen pharmazeutisch verträglichen Träger umfasst.

12. Pharmazeutische Zusammensetzung nach Anspruch 11, wobei
(a) die ASCs durch ein Verfahren erhalten werden, welches das Kultivieren von Zellen, die aus der Plazenta stammen, in einer dreidimensionalen (3D) Kultur umfasst;
(b) die ASCs positiv für mindestens einen Marker sind, der ausgewählt ist aus CD73, CD90, CD29, D7-FIB und CD105, wobei die ASCS von jedem der mindestens zwei Spender optional positiv für den mindestens einen Marker sind;
(c) die ASCs negativ für mindestens einen Marker sind, der ausgewählt ist aus CD3, CD4, CD45, CD80, HLA-DR, CD11b, CD14, CD19, CD34, CD200, KDR, CD31 und CD79, wobei die ASCs von jedem der mindestens zwei Spender optional negativ für den mindestens einen Marker sind;
(d) die ASCs PLX oder PLX-C Zellen sind;
(e) die ASCs ASCs von mindestens drei, mindestens vier, mindestens fünf, mindestens zehn, mindestens fünfundzwanzig oder mindestens 100 Spendern umfassen;
(f) die mindestens zwei Spender mindestens zwei unterschiedliche HLA Genotypen aufweisen, wobei die mindestens zwei unterschiedlichen HLA Genotypen optional Genotypen mindestens eines der HLA-A, HLA-B, HLA-DR und HLA-DQ Loci sind;
(g) die ASCs durch ein Verfahren erhalten werden, welches das Kultivieren von Zellen, die aus der Plazenta stammen, in einer zweidimensionalen (2D) Kultur umfasst; und/oder
(h) der pharmazeutisch verträgliche Träger eine isotonische Lösung ist.

13. Pharmazeutische Zusammensetzung nach Anspruch 12(a), wobei das 3D Kultivieren das Kultivieren in einem 3D Bioreaktor umfasst.

14. ASCs zur Verwendung nach Anspruch 4 oder pharmazeutische Zusammensetzung nach Anspruch 13, wobei
(a) Zellen im 3D Bioreaktor unter Perfusion kultiviert werden; und/oder
(b) der 3D Bioreaktor mindestens ein adhärentes Material umfasst, ausgewählt aus einem Polyester und einem Polypropylen.

15. Pharmazeutische Zusammensetzung nach den Ansprüchen 12(a), 13 oder 14, wobei
(i) sich das 3D Kultivieren über mindestens drei Tage erstreckt; und/oder
(ii) der 3D Kulturschritt stattfindet, bis sich mindestens 10% der Zellen teilen.

16. Pharmazeutische Zusammensetzung nach Anspruch 12(h), wobei die isotonische Lösung ferner etwa 5% menschliches Serumalbumin umfasst, wobei die isotonische Lösung optional ferner etwa 5% bis etwa 10% Dimethylsulfoxid umfasst.

17. ASCs zur Verwendung nach einem der Ansprüche 1-10 und 14 oder pharmazeutische Zusammensetzung nach einem der Ansprüche 11-16, wobei die ASCs drei Allele an jedem der HLA-A und HLA-B Loci aufweisen.

18. ASCs zur Verwendung nach einem der Ansprüche 1-10 und 14 oder pharmazeutische Zusammensetzung nach einem der Ansprüche 11-16, wobei die ASCs sowohl von mütterlichen als auch fötalen Anteilen der Plazenta erhalten werden.

19. Pharmazeutische Zusammensetzung nach einem der Ansprüche 11-18, wobei die Zusammensetzung zur Verabreichung durch intravenöse, intramuskuläre oder subkutane Injektion geeignet ist.

## Revendications

1. Cellules stromales adhérentes (CSA) dérivées de placenta pour leur utilisation en thérapie, dans lesquelles les CSA comprennent des CSA d'au moins deux placentas donneurs.

2. Cellules stromales adhérentes (CSA) dérivées de placenta pour leur utilisation dans le traitement d'une condition qui peut être traitée par l'administration de CSA à un sujet le nécessitant, dans lesquelles les CSA comprennent des CSA d'au moins deux placentas donneurs, et dans lesquelles l'affection est sélectionnée parmi une déficience en cellules souches, une cardiopathie, un trouble neurodégénératif, un cancer, un accident vasculaire cérébral, les brûlures, une perte de tissu, une perte de sang, l'anémie, une maladie auto-immune, une ischémie, la régénération des muscles squelettiques, une douleur neuropathique, un système hématopoïétique compromis, des maladies gériatriques, une affection médicale nécessitant une régénération et/ou une réparation des tissus conjonctifs.

3. CSA pour leur utilisation selon la revendication 1 ou 2, dans lesquelles
(a) les CSA sont obtenues par un procédé comprenant la mise en culture de cellules dérivées de placenta dans une culture en trois dimensions (3D) ; et/ou
(b) dans lesquelles les CSA sont positives pour au moins un marqueur sélectionné parmi CD73, CD90, CD29, D7-FIB et CD105.

4. CSA pour leur utilisation selon la revendication 3, dans lesquelles
(a) la culture en 3D comprend la culture dans un bioréacteur en 3D ;
(b) la culture en 3D a lieu pendant au moins trois jours ; et/ou
(c) l'étape de culture en 3D a lieu jusqu'à ce qu'au moins 10 % des cellules soient en prolifération.

5. CSA pour leur utilisation selon la revendication 3(b), dans lesquelles les CSA de chacun d es deux donneurs ou plus sont positives pour le ou les marqueurs.

6. CSA pour leur utilisation selon l'une quelconque des revendications 1 à 5, dans lesquelles
(a) les CSA sont négatives pour au moins un marqueur sélectionné parmi CD3, CD4, CD45, CD80, HLA-DR, CD11b, CD14, CD19, CD34, CD200, KDR, CD31 et CD79, les CSA de chacun des deux donneurs ou plus étant facultativement négatives pour le ou les marqueurs ;
(b) les CSA sont des cellules PLX ou PLX-C ;
(c) le trouble neurodégénératif est sélectionné parmi la sclérose en plaques (SP), la maladie d'Alzheimer, et la maladie de Parkinson ;
(d) l'ischémie est une maladie des artères périphériques (MAP) ;
(e) l'ischémie comprend l'ischémie du système nerveux central (SNC) ;
(f) l'ischémie est sélectionnée parmi une maladie des artères périphériques, une maladie vasculaire ischémique, une cardiopathie ischémique, une maladie cérébrale ischémique, une maladie rénale ischémique et un placenta ischémique ;
(g) l'affection médicale nécessitant une régénération et une réparation du tissu conjonctif est sélectionnée parmi une fracture osseuse, un cancer des os, une plaie de brûlure, un défaut du cartilage articulaire et une plaie profonde ;
(h) l'affection médicale nécessitant une régénération et une réparation de tissu conjonctif est sélectionnée parmi un kyste osseux sous-chondral, une fracture osseuse, l'ostéoporose, l'arthrose, une dégénérescence osseuse, un cancer, une lésion du cartilage, un défaut du cartilage articulaire, une maladie discale dégénérative, une ostéogenèse imparfaite (OI), une brûlure, une plaie de brûlure, une brûlure profonde, une cicatrisation retardée, une lésion tendineuse et une lésion ligamentaire ;
(i) la maladie auto-immune est sélectionnée parmi la polyarthrite rhumatoïde, la spondylarthrite ankylosante, l'affection abdominale inflammatoire (AAI), la SP, le diabète de type I, le syndrome de Goodpasture, la maladie de Grave, la maladie de Hashimoto, le lupus, la myasthénie gravis, le psoriasis, et le syndrome de Sjorgen, l'affection abdominale inflammatoire étant sélectionnée parmi la maladie de Crohn et la rectocolite hémorragique ;
(j) les CSA administrées comprennent des CSA d'au moins trois, d'au moins quatre, au moins cinq, d'au moins dix, d'au moins vingt-cinq, ou d'au moins 100 donneurs ;
(k) les deux donneurs ou plus ont au moins deux génotypes HLA différents, dans lesquels les deux génotypes HLA différents sont facultativement des génotypes d'au moins l'un des loci HLA-A, HLA-B, HLA-DR, et HLA-DQ ;
(l) les CSA sont administrées au sujet en une session de traitement, deux sessions de traitements, pas plus de dix sessions de traitement, ou en dix sessions de traitement ou plus ;
(m) les CSA sont administrées au cours de la vie du sujet ;
(n) les CSA de deux donneurs ou plus sont administrées au sujet à partir d'au moins une aliquote comprenant des CSA de chacun des deux donneurs ou plus, dans lequel l'aliquote comprenant des CSA de chacun des deux donneurs ou plus est facultativement préparée par un procédé comprenant au moins une étape sélectionnée parmi le mélange de cellules dérivées du placenta avant la culture in vitro, le mélange de cellules dérivées de placenta durant la culture en 2D, le mélange de cellules dérivées de placenta après la culture en 2D, le mélange de cellules dérivées de placenta durant la culture en 3D, et le mélange de cellules dérivées de placenta après la culture en 3D ;
(o) les CSA des deux donneurs ou plus sont administrées à un sujet à partir d'aliquotes comprenant chacune des CSA d'un seul donneur, dans lequel l'administration des CSA des aliquotes des deux donneurs ou plus a facultativement lieu pendant 24 heures ;
(p) les CSA sont administrées pendant au moins une session de traitement, la ou les sessions de traitement comprennent l'administration de 1 à 40, de 5 à 40, de 10 à 30, d'environ 5, d'environ 10, d'environ 15, d'environ 20, d'environ 25, d'environ 30, d'environ 35, d'environ 40, ou d'environ 45, ou d'environ 50 injections séparées au sujet, le sujet nécessitant facultativement un traitement pour une ischémie critique des membres et les CSA sont facultativement administrées au sujet par environ 30 à environ 50 injections intramusculaires pendant une ou deux sessions de traitement ; et/ou
(q) les CSA sont obtenues par un procédé comprenant la mise en culture de cellules dérivées de placenta dans une culture en deux dimensions (2D).

7. CSA pour leur utilisation selon la revendication 6(d), dans lequel la MAP est une ischémie des membres (IM).

8. CSA pour leur utilisation selon la revendication 7, dans lequel l'ischémie des membres est une ischémie critique des membres (ICM).

9. CSA pour leur utilisation selon l'une quelconque des revendications 1 à 5, dans lesquelles le tissu conjonctif comprend au moins un l'un d'un tendon, d'un os et d'un ligament.

10. CSA pour leur utilisation selon l'une quelconque des revendications 1 à 5, dans lesquelles le système hématopoïétique compromis est le résultat d'une radiothérapie ou d'une chimiothérapie.

11. Composition pharmaceutique comprenant des cellules stromales adhérentes (CSA), dans laquelle la composition pharmaceutique comprend des CSA d'au moins deux placentas donneurs et un vecteur pharmaceutiquement acceptable.

12. Composition pharmaceutique selon la revendication 11, dans laquelle
(a) les CSA sont obtenues par un procédé comprenant la mise en culture de cellules dérivées de placenta dans une culture en trois dimensions (3D) ;
(b) les CSA sont positives pour au moins un marqueur sélectionné parmi CD73, CD90, CD29, D7-FIB et CD105, les CSA de chacun des deux donneurs ou plus étant facultativement positives pour le ou les marqueurs ;
(c) les CSA sont négatives pour au moins un marqueur sélectionné parmi CD3, CD4, CD45, CD80, HLA-DR, CD11b, CD14, CD19, CD34, CD200, KDR, CD31 et CD79, les CSA de chacun des deux donneurs ou plus étant facultativement négatives pour le ou les marqueurs ;
(d) les CSA sont des cellules PLX ou PLX-C ;
(e) les CSA administrées comprennent des CSA d'au moins trois, d'au moins quatre, au moins cinq, d'au moins dix, d'au moins vingt-cinq, ou d'au moins 100 donneurs ;
(f) les deux donneurs ou plus ont au moins deux génotypes HLA différents, les deux génotypes HLA différents étant facultativement des génotypes d'au moins l'un des loci HLA-A, HLA-B, HLA-DR, et HLA-DQ ;
(g) les CSA sont obtenues par un procédé comprenant la mise en culture de cellules dérivées de placenta dans une culture en deux dimensions (2D) ; et/ou
(h) le vecteur pharmaceutiquement acceptable est une solution isotonique.

13. Composition pharmaceutique selon la revendication 12(a), dans laquelle la culture en 3D comprend la culture dans un bioréacteur en 3D.

14. CSA pour leur utilisation selon la revendication 4, ou la composition pharmaceutique selon la revendication 13, dans lesquelles
(a) les cellules dans le bioréacteur en 3D sont cultivées sous perfusion ; et/ou
(b) le bioréacteur en 3D comprend au moins un matériau adhérent sélectionné parmi un polyester et un polypropylène.

15. Composition pharmaceutique selon la revendication 12(a), 13 ou 14, dans laquelle
(i) la culture en 3D a lieu pendant au moins trois jours ; et/ou
(ii) l'étape de culture en 3D a lieu jusqu'à ce qu'au moins 10 % des cellules soient en prolifération.

16. Composition pharmaceutique selon la revendication 12(h), dans laquelle la solution isotonique comprend en outre environ 5 % de sérum albumine humaine, dans laquelle la solution isotonique comprend facultativement en outre environ 5 % à environ 10 % de diméthyl sulfoxyde.

17. CSA pour leur utilisation selon l'une quelconque des revendications 1 à 10, et 14 ou la composition pharmaceutique selon l'une quelconque des revendications 11 à 16, dans laquelle les CSA ont trois allèles au niveau de chaque des loci HLA-A et HLA-B.

18. CSA pour leur utilisation selon l'une quelconque des revendications 1 à 10, et 14, ou la composition pharmaceutique selon l'une quelconque des revendications 11 à 16, dans lesquelles les CSA sont obtenues de parties maternelles et foetales du placenta.

19. Composition pharmaceutique selon l'une quelconque des revendications 11 à 18, dans laquelle la composition est adaptée à une administration par injection intraveineuse, intramusculaire ou sous-cutanée.
